(19) Europäisches Patentamt / European Patent Office / Office européen des brevets

(11) **EP 4 347 790 B1**

(12) **EUROPEAN PATENT SPECIFICATION**

(45) Date of publication and mention of the grant of the patent:
**22.10.2025 Bulletin 2025/43**

(21) Application number: **22726528.7**

(22) Date of filing: **18.05.2022**

(51) International Patent Classification (IPC):
*C12N 5/00* (2006.01)     *C12N 5/10* (2006.01)
*C12N 5/078* (2010.01)     *A61K 38/45* (2006.01)
*C12N 9/10* (2006.01)     *C12N 5/071* (2010.01)

(52) Cooperative Patent Classification (CPC):
**C12N 5/10; A61K 35/18; A61P 3/00; A61P 25/28;
C12N 5/067; C12N 5/0676; C12N 9/1007;
C12N 9/1085; C12Y 201/01002; C12Y 205/01006;**
A61K 38/00; C07K 2319/21; C07K 2319/95;
C12N 5/0641; C12N 2500/32;          (Cont.)

(86) International application number:
**PCT/IB2022/054607**

(87) International publication number:
**WO 2022/248975 (01.12.2022 Gazette 2022/48)**

(54) **ENGINEERING NEW METABOLIC PATHWAYS IN ISOLATED CELLS FOR THE DEGRADATION OF GUANIDINOACETIC ACID AND SIMULTANEOUS PRODUCTION OF CREATINE**

MANIPULATION NEUER STOFFWECHSELWEGE IN ISOLIERTEN ZELLEN ZUM ABBAU VON GUANIDINOESSIGSÄURE UND ZUR SIMULTANEN HERSTELLUNG VON KREATIN

INGÉNIERIE DE NOUVELLES VOIES MÉTABOLIQUES DANS DES CELLULES ISOLÉES POUR LA DÉGRADATION DE L'ACIDE GUANIDINOACÉTIQUE ET PRODUCTION SIMULTANÉE DE CRÉATINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.05.2021 IT 202100013814**

(43) Date of publication of application:
**10.04.2024 Bulletin 2024/15**

(73) Proprietor: **Quince Therapeutics S.p.A.**
**20091 Bresso MI (IT)**

(72) Inventors:
• **ROSSI, Luigia**
**61029 Urbino PU (IT)**
• **MAMBRINI, Giovanni**
**41037 Mirandola MO (IT)**
• **ALIANO, Mattia Paolo**
**61010 Tavullia PU (IT)**
• **BIANCHI, Marzia**
**61034 Fossombrone PU (IT)**

• **PIERIGÈ, Francesca**
**61029 Urbino PU (IT)**
• **MAGNANI, Mauro**
**61029 Urbino PU (IT)**

(74) Representative: **Di Giovine, Paolo et al**
**Società Italiana Brevetti S.p.A.**
**Piazza di Pietra, 38-39**
**00186 Roma (IT)**

(56) References cited:
**WO-A1-2020/081994**

• **ALMEIDA L S ET AL: "Overexpression of GAMT restores GAMT activity in primary GAMT-deficient fibroblasts", MOLECULAR GENETICS AND METABOLISM, ACADEMIC PRESS, AMSTERDAM, NL, vol. 89, no. 4, 1 December 2006 (2006-12-01), pages 392 - 394, XP024947183, ISSN: 1096-7192, [retrieved on 20061201], DOI: 10.1016/J.YMGME.2006.06.006**

(Cont. next page)

- LEUZZI VINCENZO ET AL: "Erythrocyte-mediated delivery of recombinant enzymes", JOURNAL OF INHERITED METABOLIC DISEASE, KLUWER, DORDRECHT, NL, vol. 39, no. 4, 30 March 2016 (2016-03-30), pages 519 - 530, XP035989917, ISSN: 0141-8955, [retrieved on 20160330], DOI: 10.1007/S10545-016-9926-0
- "Erythrocytes loaded with recombinant proteins as enzyme replacement therapy for the treatment of rare diseases", 24 June 2021, article ALIANO MATTIA PAOLO: "Erythrocytes loaded with recombinant proteins as enzyme replacement therapy for the treatment of rare diseases", pages: 1 - 79, XP055884226
- BIANCHI MARZIA ET AL: "Engineering new metabolic pathways in isolated cells for the degradation of guanidinoacetic acid and simultaneous production of creatine", MOLECULAR THERAPY- METHODS & CLINICAL DEVELOPMENT, vol. 25, 1 June 2022 (2022-06-01), GB, pages 26 - 40, XP055953065, ISSN: 2329-0501, DOI: 10.1016/j.omtm.2022.02.007

Remarks:
The complete document including Reference Table(s) and the Sequence Listing(s) can be downloaded from the EPO website

(52) Cooperative Patent Classification (CPC): (Cont.)
C12N 2500/34

Remarks:

**Description**

**Technical field**

The present invention relates to an isolated cell modified for catalyzing the conversion of guanidinoacetate acid (GAA) into creatine in the presence of glucose and methionine using an exogenous guanidinoacetate methyltransferase (GAMT) and methionine adenosyltransferase (MAT) protein, a pharmaceutical composition comprising a plurality of said isolated cells and uses thereof.

**Background**

Guanidinoacetate methyltransferase (GAMT) deficiency was recognized as the first autosomal recessive inborn error of creatine (Cr) metabolism in 1994 and is part of the Cerebral Creatine Deficiency Syndromes (CCDS). GAMT deficiency has an incidence of 1:250.000 newborns (Mercimek-Mahmutoglu S & Salomons GS, 2009) and a prevalence of <1/1.000.000 (www.orpha.net). This enzyme catalyzes the last step of Cr synthesis, facilitating the transfer of a single methyl group from S-adenosyl methionine (SAM) to guanidinoacetate acid (GAA), forming Cr and S-adenosylhomocysteine (adoHcys). Cr, a molecule of vital importance for the energy of the organism, is released into the blood and captured by the tissues that require it (Iqbal F, 2015). At the biochemical level, the disease is characterized by extremely low levels of Cr in the blood and tissues and by the accumulation of its precursor GAA, a highly toxic molecule for the organism, especially for the brain (Iqbal F, 2015). All CCDS share a common set of symptoms that reflect the imbalance of mental functions such as mental retardation of various degrees, severe lack of language skills, and delay in their acquisition, behavioral disorders that may also fall within the spectrum of autism. Ultimately, alterations in movement capabilities such as dystonia, dyskinesia, ataxia, and catatonia (Mercimek-Mahmutoglu S & Salomons GS, 2009; Iqbal F, 2015; Clark JF & Cecil KM, 2015) might be present. The only available therapy for the treatment of subjects with this disease is high dose Cr supplementation, often accompanied by administration of ornithine and/or sodium benzoate and restriction of arginine with the goal of restoring the normal Cr level in the brain and, at the same time, avoid GAA accumulation. However, to be successful, therapies must be strictly followed, which can often lead to poor compliance of patients and their families, thus compromising the outcome of the treatment itself.

Within this context, there is therefore an urgent need for novel therapeutic approaches that could enable an efficient treatment of subjects suffering from a deficit of Cr.

**Summary of the invention**

Because of the severity of the GAMT deficiency and the absence of approved therapeutic interventions, the authors of the present invention have developed a circulating cellular bioreactor consisting in engineered cells for the degradation of guanidinoacetate acid (GAA) and simultaneous production of creatine.

The technical problem posed and solved by the present invention is hence that of providing an effective therapeutic approach for the prevention and/or treatment of a disease or condition caused by and/or characterized by a deficit of creatine, such as a GAMT deficiency.

The solution provided by the present invention is represented by an isolated cell according to claim 1, modified for catalyzing the conversion of GAA into creatine in the presence of glucose and methionine using an exogenous guanidinoacetate methyltransferase (GAMT) and methionine adenosyltransferase (MAT) protein. These two enzymes, collectively, activate a new metabolic pathway that permits said isolated cells, such as red blood cells (RBCs) to catabolize GAA with the simultaneous formation of creatine. The engineered cells are thus effective in the degradation of GAA, the toxic metabolite that accumulates in the blood of patients with a GAMT deficiency. At the same time, the engineered cells produce creatine, a metabolite that is under-represented in patients with GAMT deficiency. For these reasons, the isolated cells of the invention may be particularly useful in the treatment of subjects suffering from a disease or condition caused by and/or characterized by a deficit of creatine, such as GAMT deficiency or a methionine dependent tumor.

It is important to underline that in the present invention in order to treat a specific genetic disease the inventors do not use a common enzyme replacement therapy, but for the first time they modified a full metabolic pathway in the cells.

In one particular embodiment, the invention includes the expression and purification of a recombinant truncate form of human GAMT further mutagenized in the positions A26N-L37M-I187Q-V215Q and a recombinant form of human MAT (Methionine adenosyltransferase) without the presence of the regulatory subunits. The authors of the invention have demonstrated that both recombinant proteins can be encapsulated into human RBCs according to standard procedures (Magnani M et al., 1988) providing engineered RBCs capable of catalyzing the conversion of GAA into creatine in the presence of glucose and methionine at physiological blood concentrations. Advantageously, said engineered RBCs could be useful in the treatment of patients with a GAMT deficiency and/or to increase the endogenous production of creatine. These engineered RBCs can be prepared *ex vivo* and re-infused back in the original donor or a compatible patient in need.

In some embodiments, the present application particularly describes (i) a recombinant human GAMT fully modified to be catalytically active, stable within human red blood cells and to be easily produced, and (ii) a recombinant human MAT where the regulatory domains have been removed to produce a small, stable, catalytically active protein and to be easily produced, as well as (iii) an efficient method for the encapsulation of said recombinant proteins into isolated cells, such as RBCs.

RBCs loaded with recombinant enzymes have been previously described (Leuzzi V et al, 2016) and selected constructs are in clinical development (Rossi L et al, 2020). In this invention, an entire metabolic pathway can be engineered by only two recombinant engineered human enzymes that, taking advantage of some endogenous properties of human RBCs, utilize extracellular GAA and continuously release Cr. Furthermore, the modified cells, such as the RBCs, maintain their native properties in terms of morphology, biochemistry and functions.

Hence, objects of the present invention are:

- An isolated cell modified for catalyzing the conversion of guanidinoacetate acid (GAA) into creatine in the presence of glucose and methionine using an exogenous guanidinoacetate methyltransferase (GAMT) and methionine adenosyltransferase (MAT) protein.
- An isolated cell according to any of the embodiments described in the present specification and in the claims for use as a medicament, in particular for use in the treatment of the GAMT deficiency.
- A pharmaceutical composition comprising a plurality of modified cells according to any of the embodiments described in the present specification and in the claims and one or more carriers and/or diluent.
- An *in vitro* method for the preparation of a modified cell comprising a step of modifying a cell whereby said cell is capable of catalyzing the conversion of guanidinoacetate acid (GAA) into creatine in the presence of glucose and methionine using an exogenous guanidinoacetate methyltransferase (GAMT) and methionine adenosyltransferase (MAT) protein.

Additional advantages and/or embodiments of the present invention will be evident from the following detailed description.

**Brief description of the drawings**

The present invention and the following detailed description of preferred embodiments thereof may be better understood with reference to the following figures:

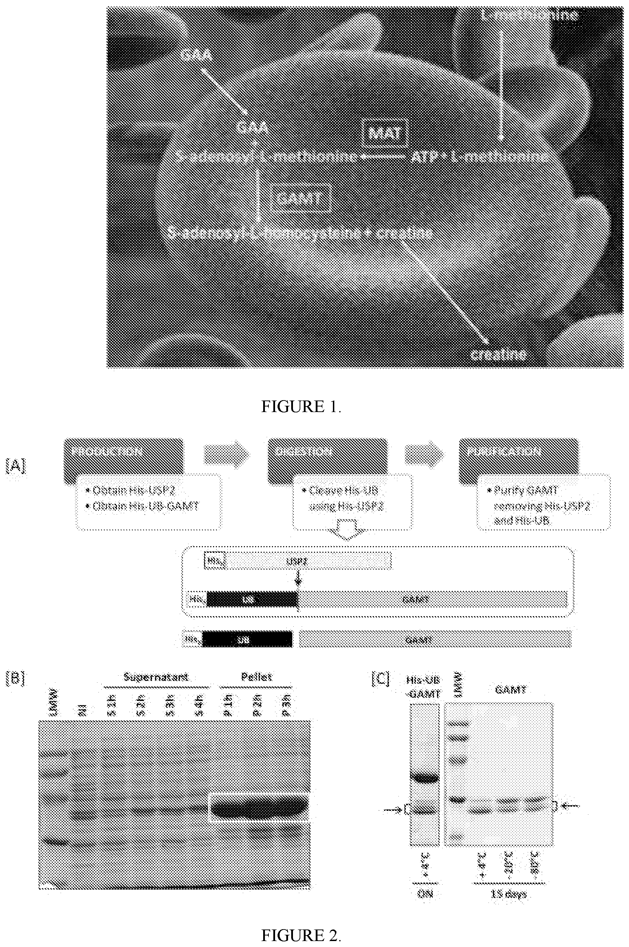

**Figure 1:** Schematic representation of engineering a multi metabolic pathway in human RBCs.

**Figure 2:** Expression strategy of native GAMT - [A] Expression strategy to obtain NO-tag GAMT from the pET45b/His-UB-GAMT construct. [B] SDS-PAGE of a representative time-course induction experiment, where Supernatant (S) and Pellet (P) fractions are compared; NI is the not-induced sample. [C] Stability assay: His-UB-GAMT and NO-tag GAMT were stored at different temperatures, for the times indicated. ON, overnight. LMW, low molecular weight protein standard.

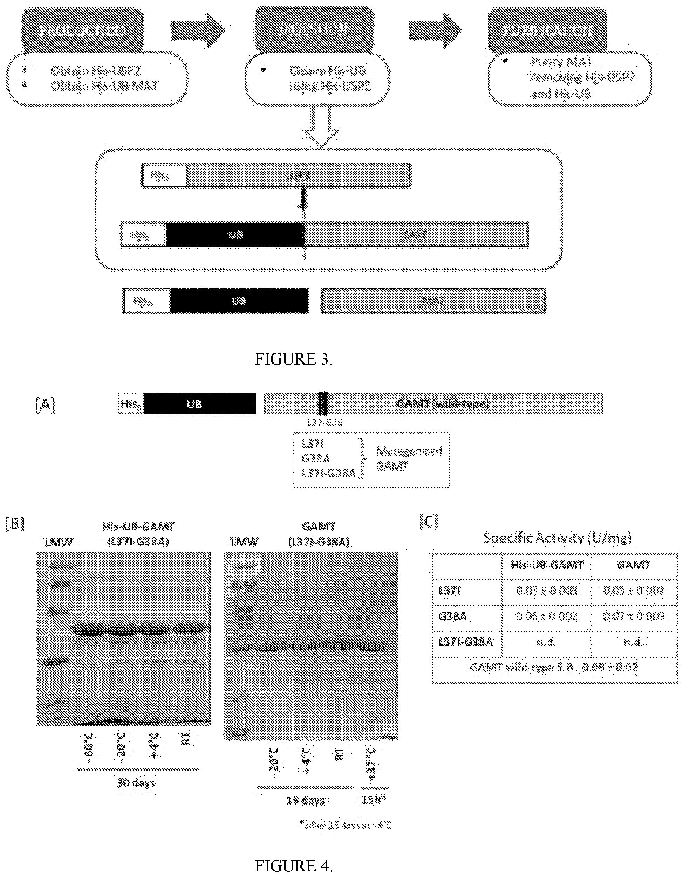

**Figure 3:** Expression strategy to obtain NO-tag MAT from the pET45b/His-UB-MAT construct.

**Figure 4:** GAMT mutagenesis and protein stability. - [A] His-UB-GAMT expression cassette: the designed amino acid substitutions within GAMT coding sequence are highlighted. [B] Stability assay: His-UB-GAMT and NO-tag GAMT carrying the double amino acid substitutions (L37I-G38A) were stored at different temperatures, for the times indicated. LMW, low molecular weight protein standard. RT, room temperature. [C] Specific activity of the three mutagenized GAMT, both in the precursor and tag-less form.

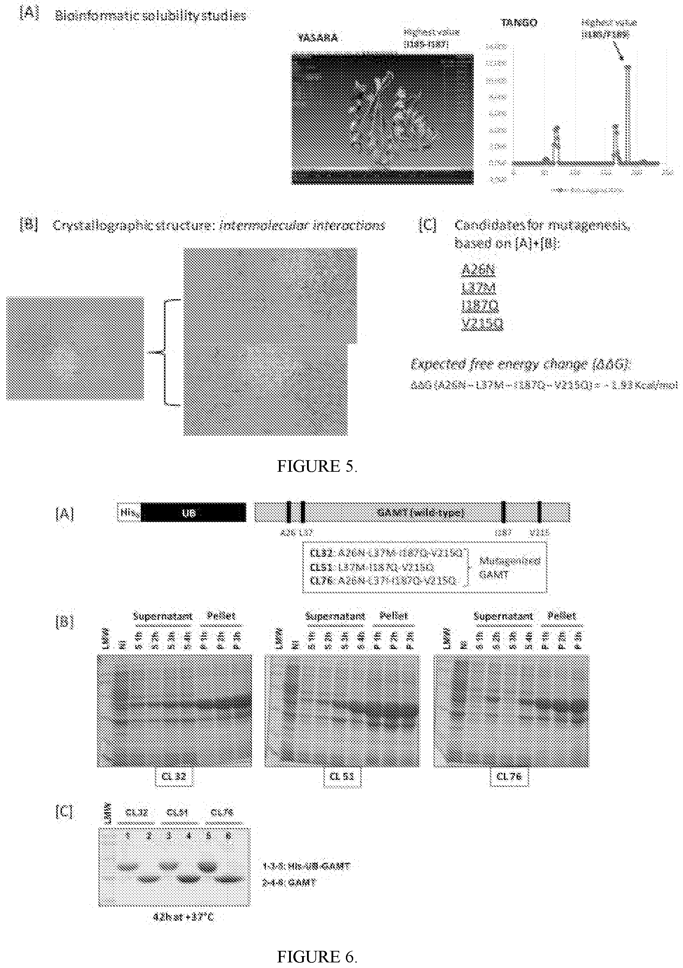

**Figure 5:** GAMT engineering to increase protein solubility and stability. - [A] Bioinformatic solubility studies with YASARA and TANGO tools. [B] In silico analysis of GAMT intermolecular interactions, based on the Crystallographic structure. Changed amino acids are in blue. [C] Summary of the candidate residues for mutagenesis and calculation of the expected free energy change ($\Delta\Delta G$).

**Figure 6:** Mutagenesis to increase GAMT solubility and stability. - [A] His-UB-GAMT expression cassette: the designed amino acid substitutions within GAMT coding sequence are highlighted. The mutagenesis carried by the three selected clones (CL32, CL51, CL76) are shown. [B] SDS-PAGE of a representative time-course induction experiment, for each mutagenized construct, where Supernatant and Pellet fractions are compared; NI is the not-induced sample. [C] Stability assay: His-UB-GAMT and NO-tag GAMT carrying the amino acid substitutions indicated in [A] were stored at 37°C for 42 h. LMW, low molecular weight protein standard.

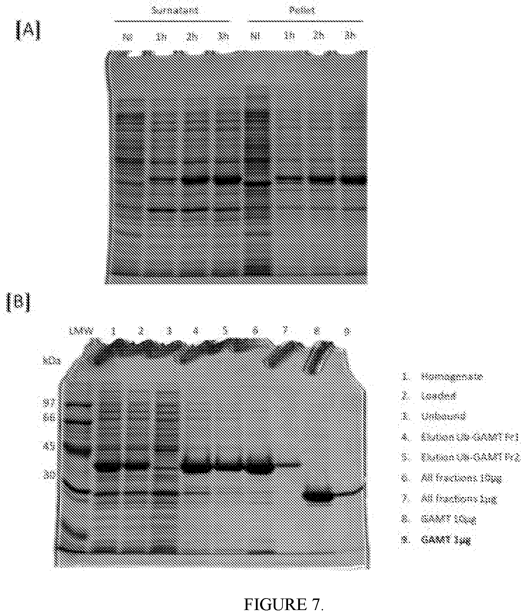

**Figure 7:** Scaling-up induction and purification of GAMT CL32. - [A] Time-course induction of the mutagenized construct CL32 (in 5.5 L LB, using the Bioreactor). Supernatant and Pellet fractions are resolved by SDS-PAGE; NI is the not-induced sample. [B] Purification of His-UB-GAMT CL32 by immobilized metal ion affinity chromatography (IMAC), with the Akta Purifier. LMW, low molecular weight protein standard.

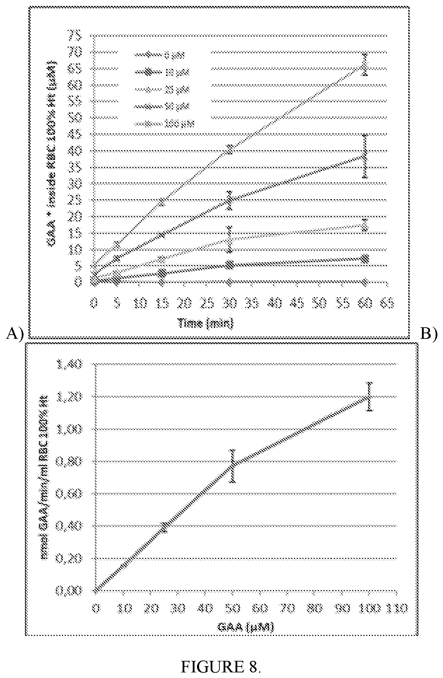

**Figure 8:** GAA uptake by human RBCs. - [A] RBCs were incubated for 1 h at 37°C in the presence of different $^{13}C_2$ GAA (GAA*) amounts (0-100 $\mu$M). At different incubation times, GAA* concentration inside RBCs was evaluated by ESI-MS/MS analyses of dried RBC spots. Values are mean $\pm$ SD of 3 separate experiments. [B] Velocity of GAA* uptake inside RBCs.

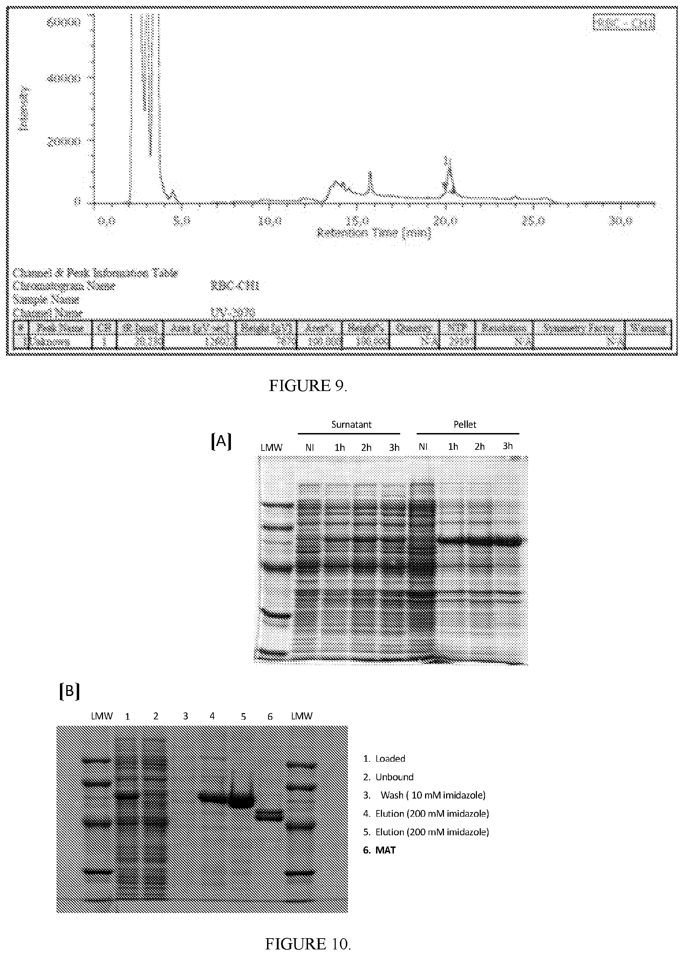

**Figure 9:** SAM chromatogram of RBC sample. - Representative chromatogram of RBC SAM content of packed RBCs. The retention time for SAM was 20 min.

**Figure 10:** Scaling-up induction and purification of MAT - [A] Time-course induction of the pET45b-UB_MAT2A construct (in 5.5 liters Synthetic medium, using the Bioreactor). Supernatant and Pellet fractions are resolved by SDS-PAGE; NI is the not-induced sample. [B] Purification of His-UB-MAT by immobilized metal ion affinity chromatography (IMAC). The final MAT product obtained by DEAE chromatography, with the Akta Purifier, was loaded in lane 6. LMW, low molecular weight protein standard.

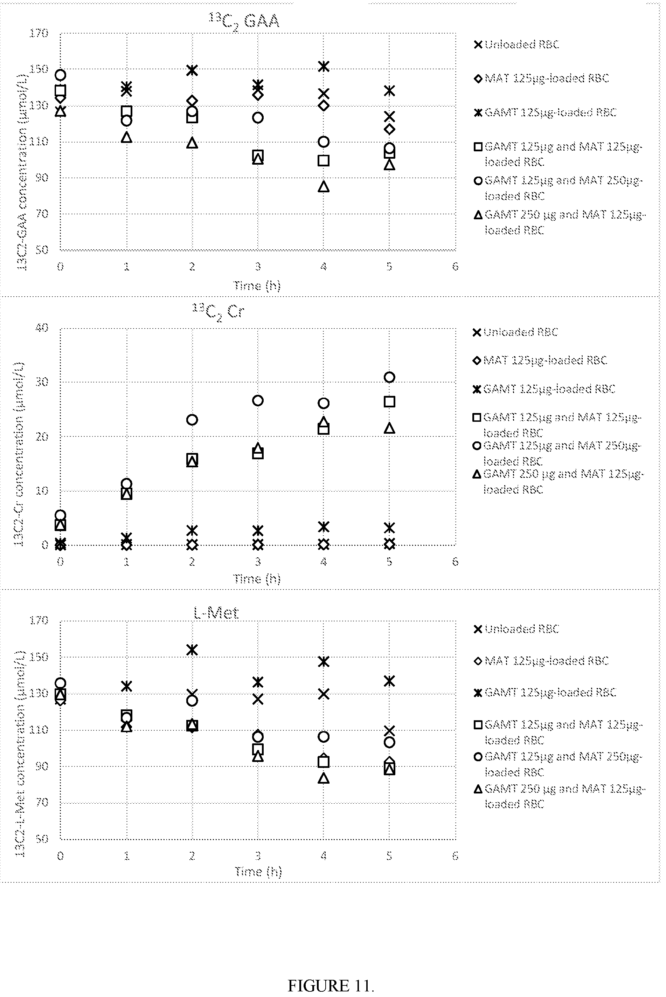

**Figure 11:** GAA consumption [A], Cr production [B] and L-Met [C] consumption by loaded RBCs. - GAA, Cr and L-Met values after incubation of GAMT and/or MAT loaded RBC suspensions in phosphate buffer saline solution at 40% Ht in the presence of 200 $\mu$M 13C2 GAA and 200 $\mu$M L-Met for 21 hours at 37°C. At the planned time points, biochemical monitoring of 13C2 GAA, 13C2 Cr and L-Met by MS/MS analysis of RBC suspensions were performed. In the inset, the different loading conditions are specified.

## Detailed description

In the following, several embodiments of the invention will be described. It is intended that the features of the various embodiments can be combined, where compatible. In general, subsequent embodiments will be disclosed only with respect to the differences with the previously described ones.

As previously mentioned, a first object of the present invention is represented by an isolated cell modified for catalyzing the conversion of guanidinoacetate acid (GAA) into creatine in the presence of glucose and methionine using an exogenous guanidinoacetate methyltransferase (GAMT) and methionine adenosyltransferase (MAT) protein.

As used herein, the expression "catalyzing the conversion" means the biological transformation of GAA into creatine in the presence of glucose and methionine, for example at physiological blood concentrations thanks to enzymes that act as catalysts. In particular, the enzymes that are exploited in the present invention are guanidinoacetate methyltransferase (GAMT) and methionine adenosyltransferase (MAT) proteins.

Guanidinoacetate N-methyltransferase (GAMT) (EC 2.1.1.2) is an enzyme that catalyzes the chemical reaction and is encoded by gene *GAMT,* located on chromosome 19p13.3. The two substrates of this enzyme are S-adenosyl methionine and guanidinoacetate, whereas its two products are S-adenosylhomocysteine and creatine.

This enzyme belongs to the family of transferases, specifically those transferring one-carbon group methyltransferases. This enzyme participates in glycine, serine and threonine metabolism and arginine and proline metabolism.

The protein encoded by this gene is a methyltransferase that converts guanidinoacetate to creatine, using S-adenosylmethionine as the methyl donor. Defects in this gene have been implicated in neurologic syndromes and muscular hypotonia, probably due to creatine deficiency and accumulation of guanidinoacetate in the brain of affected individuals.

S-adenosylmethionine synthetase (MAT) (EC 2.5.1.6), also known as methionine adenosyltransferase (MAT), is an enzyme that creates S-adenosylmethionine (a.k.a. AdoMet, SAM or SAMe) by reacting methionine (a non-polar amino acid) and ATP (the basic currency of energy).

In an embodiment of the invention herein described, according to any one of the embodiments disclosed, the isolated cell is a human cell, which could be selected from erythrocytes, hepatocytes, pancreas cells, kidney epithelial cells, human brain cells.

In another embodiment, the isolated cell lacks or exhibits a reduced endogenous GAMT and/or MAT enzymatic activity, for example, said cells are not able to encode correct GAMT and/or MAT proteins.

The expression "reduced endogenous GAMT and/or MAT enzymatic activity", as used herein, refers to a cell which exhibits endogenous GAMT and/or MAT enzymatic activity that is diminished as compared with the activity of endogenous GAMT and/or MAT as determined for a cell isolated from a control organism, such as a cell isolated from a healthy subject.

The term "endogenous" refers to a referenced molecule (e.g., nucleic acid) or activity already present in the host cell prior to a particular genetic modification (e.g., a genetic composition, trait, or biosynthetic activity of a wild-type cell or a parent cell).

In a further embodiment of this invention, according to any one of the embodiments herein disclosed, the cell is isolated from a subject suffering of Guanidinoacetate methyltransferase (GAMT) deficiency. In the case the human cell would be an erythrocyte, the starting erythrocytes can be obtained by taking and isolating the red blood cells from an individual's blood sample. Preferably, the starting sample is treated with an anti-coagulant agent, for example heparin, in order to avoid coagulation. Optionally, before being subjected to the treatment according to the invention, the erythrocytes can be isolated and subjected to one or more washes with physiological solution in order to obtain a population of starting erythrocytes in which they are not present, or are present in negligible concentrations, any contaminants, for example, plasma, platelets, lymphocytes etc. In one embodiment, the cell is isolated as described in the following reference (ITRM2013061A1).

In another embodiment of the invention, in accordance with any one of the embodiments herein disclosed, the cell is loaded with said GAMT protein, a fragment or a variant thereof, and with said MAT protein, a fragment or a variant thereof.

The expression "is loaded with" refers to a process through which GAMT protein, a fragment or a variant thereof, and MAT protein, a fragment or a variant thereof, are inserted into the host cells, preferably erythrocytes. An example of said process is described in-depth in patent US 10,849,858.

The word "fragment" refers to short segments of the peptide backbone, typically from 5 to 15 residues long, and do not include the side chains.

The word "variant" refers to a member of a set of highly similar proteins that originate from a single gene or gene family and are the result of genetic differences. While many perform the same or similar biological roles, some variants have unique functions.

Forms part of the present invention are the proteins, whose sequences are: SEQ. ID NO 2, SEQ. ID NO 3 and SEQ ID NO 4.

In a further embodiment of this invention, according to any one of the embodiments herein disclosed, the isolated cell is transformed or transfected with exogenous nucleic acids comprising at least a nucleic acid sequence encoding said GAMT protein, a fragment or a variant thereof, and a nucleic acid sequence encoding at least said MAT protein, a fragment or a variant thereof.

The word "transformed" is referred to a host cell that has undergone a transformation process. Transformation is, simply, the process of altering a cell's genetic code through the uptake of foreign DNA from the environment. Plasmid transformation is used to describe the (non-viral) horizontal gene transfer of plasmids between bacteria.

The term "transfected" means that a host cell is subjected to transfection. Transfection is the process of introducing exogenous biological material into eukaryotic cells, in most cases mammalian cells. It is most common to insert genetic material, usually including DNA and siRNA, but, in general, proteins (such as antibodies) can also be transfected. The process and methods for carrying it out are similar to those for bacterial transformation, but this involves bacteria and sometimes plant cells. The transfection process can be done: *in vitro* (on target cells in long-term cell cultures), *ex vivo* (on cells isolated from an organism and transferred to culture medium).

Transfection can generally be done in two ways: either by overexpressing the gene in question or by silencing it. In the first case, a higher than normal production of the gene product is induced by inserting additional copies of the same gene, for example. In the second case, the gene will be 'blocked'.

The term "exogenous" refers to introduction of a referenced molecule (e.g., nucleic acid such as DNA or mRNA, but also the protein) or referenced activity into a host cell. A nucleic acid may exogenously be introduced into a host in any suitable manner. For example, a nucleic acid can be introduced into a host cell and inserted into a host chromosome, or the nucleic acid can be introduced into the host as non-chromosomal genetic material, such as a vector (e.g., a plasmid) that does not integrate into the host chromosome. A nucleic acid encoding a protein may be introduced in an expressible form (i.e., so that the nucleic acid can be transcribed and translated). An exogenous "activity" (e.g., biosynthesis activity) refers to an activity introduced into a host cell, such as by introducing one or more nucleic acids to the host that are expressed to provide the activity.

In a further embodiment of the present invention, in accordance with any one of the embodiments herein disclosed, the nucleic acid sequence encoding said GAMT protein, a fragment or a variant thereof, and said nucleic acid sequence encoding said MAT protein, a fragment or a variant thereof, are operably linked to a His-tag coding sequence.

In another embodiment, according to any one of the embodiments herein disclosed, the nucleic acid sequence encoding said GAMT protein, a fragment or a variant thereof, and the nucleic acid sequence encoding said MAT protein, a fragment or a variant thereof, further comprise a ubiquitin coding sequence downstream of said His-tag coding sequence.

In one embodiment the nucleic acid sequence encoding said GAMT protein, a fragment or a variant thereof, is selected from SEQ ID NO 6, SEQ ID NO 7, SEQ ID NO 8 or SEQ ID NO 9 and the nucleic acid sequence encoding said MAT protein, a fragment or a variant thereof, is SEQ ID NO 10 or SEQ ID NO 11.

In one aspect the present invention refers to the nucleic acid sequences SEQ ID NO 6, SEQ ID NO 7, SEQ ID NO 8, SEQ ID NO 9, SEQ ID NO 10 and SEQ ID NO 11.

In a preferred embodiment of the present invention, according to any one of the embodiments herein disclosed, the nucleic acid sequence encoding said GAMT protein, a fragment or a variant thereof, and the nucleic acid sequence encoding said MAT protein, a fragment or a variant thereof, are comprised in one or more expression vectors.

In one embodiment the present invention refers the nucleic acid sequence encoding said GAMT protein, a fragment or a variant thereof, and the nucleic acid sequence encoding said MAT protein, a fragment or a variant thereof according to any one of the embodiments herein disclosed.

The word "vector" refers to any vehicle, often a virus or a plasmid, that is used to ferry a desired DNA sequence into a host cell as part of a molecular cloning procedure. Depending on the purpose of the cloning procedure, the vector may assist in multiplying, isolating, or expressing the foreign DNA insert.

In an embodiment, the expression vector can be selected among the following ones: a plasmid, a yeast vector, a mammalian vector, a viral vector, a single-stranded phage, a double-stranded phage, an artificial chromosome and/or a combination thereof.

In a preferred embodiment, in accordance with any one of the embodiments herein disclosed, the expression vector is a plasmid, in particular pET-45b.

pET expression vectors contain the promoter recognized by the RNA polymerase of the T7 bacteriophage, the lac operon operator sequence, the T7 ribosomal binding site, a multiple cloning site and the T7 transcriptional terminator. The plasmid must be transformed into a strain capable of expressing the cloned gene, which has a copy of the sequence encoding the phage RNA polymerase. The system is designed to ensure strict control of the expression of exogenous sequences. In the absence of lactose or its analogue IPTG, the bacterial RNA polymerase cannot carry on the transcription from the lacUV5 promoter. The gene encoding the lac operon repressor is actually present in these vectors, which binds to the operator sequence, located downstream of the promoter, thus preventing the activity of the enzyme.

In particular, pET-45b plasmid has the capability to express fusion proteins with an N-terminal His-Tag coding sequence that results in native protein after the purification and cleavage process. The plasmid contains a strong T7lac promoter, an amino-terminal His-Tag coding sequence, and multiple cloning site (MCS) regions which are designed to allow the generation of target proteins with minimal vector-encoded fusion.

In another embodiment of the invention, according to any one of the embodiments herein disclosed, the expression vector is a viral vector selected from the following list: adenovirus, adeno-associated virus (AAV), lentivirus, retrovirus, cytomegalovirus (CMV), hybrids or other vectors suitable to introduce the DNA in a mammalian cell.

In further embodiment, in accordance with any one of the embodiments herein disclosed, the fragment or the variant thereof, have the same or an improved enzymatic activity compared to the wild-type protein.

The expression "improved activity" or the like refers to a detectable increase in activity of a protein or an enzyme. The term "improved activity" used herein may mean that a modified (for example, genetically engineered) protein or enzyme shows higher activity than a comparable protein or enzyme of the same type, such as a protein or an enzyme which does not have a particular genetic modification (e.g., an original or wild-type protein or enzyme, or the activity level of a protein or enzyme of a host cell that served as the starting point for genetic modification). For example, activity of a modified or engineered protein or enzyme may be higher than activity of a non-engineered protein or enzyme of the same type (e.g. a wild-type protein or an enzyme, or the activity exhibited by a protein or enzyme of a host cell) by about 5% or more, about 10% or more, about 15% or more, about 20% or more, about 30% or more, about 50% or more, about 60% or more, about 70% or more, or about 100% or more. A host cell having a protein or enzyme having an improved enzymatic activity may be verified by any methods known in the art.

The expression wild-type (WT) refers to the phenotype of the typical form of a species as it occurs in nature.

In another embodiment of the present invention, according to any one of the embodiments herein disclosed, GAMT and MAT are the naturally or recombinant human GAMT and MAT, preferably said MAT is the catalytic subunits MAT2A.

In one embodiment sequences are for example SEQ ID NO. 1, SEQ ID NO 2, SEQ ID NO 3 and SEQ ID NO 4 for GAMT and SEQ ID NO 5 for MAT, or their variants, with at least 98%, preferably 99% of identities.

The term "sequence identity" of a nucleic acid or polypeptide used herein refers to a degree of identity of bases or amino acid residues of two corresponding sequences of a particular comparable region, measured after the sequences are aligned to be matched with each other as much as possible. The sequence identity is a value that is measured by comparing optimally aligned two corresponding sequences of a particular comparable region, wherein in the comparable region, a part of the sequence may be added or deleted compared to a reference sequence. A percentage of the sequence identity may be calculated by comparing two optimally aligned corresponding sequences in an entire comparable region, determining the number of locations where an amino acid or a nucleotide is identical in the two sequences to obtain the number of matched locations, dividing the number of the matched locations by the total number (that is, a range size) of all locations within a comparable range, and multiplying the result by 100 to obtain a percentage of the sequence identity. The percent of the sequence identity may be determined by using any known sequence comparable program, and an example of the program is BLASTN and BLASTP (NCBI), CLC Main Workbench (CLC bio.), MegAlign™ (DNASTAR Inc).

In one embodiment of the present invention, said GAMT, a fragment or variant thereof, is the human GAMT wherein one or more of the following mutations A26N, L37M, L37I, I187Q and V215Q are inserted in the sequence of said protein, the amino acid numbering refers to the full-length wild-type GAMT, including the starting Methionine (M1), which is not expressed by recombinant constructs.

In one embodiment of the present invention, said GAMT protein has the sequence SEQ ID NO 1, SEQ ID NO 2, SEQ ID NO 3, SEQ ID NO 4 or SEQ ID NO 32. In another preferred embodiment, according to any one of the embodiments herein disclosed, said GAMT, a fragment or variant thereof, is the GAMT protein having the sequence SEQ ID NO 1 wherein one or more of the following mutations are inserted in the sequence of said protein: A26N, L37M, I187Q and V215Q, preferably wherein all of said mutations are inserted, wherein the amino acid numbering of said mutations refers to the full-length wild-type GAMT, including the starting Methionine (M1).

In one embodiment of the present invention, in accordance with any one of the previous embodiments, said MAT, a fragment or variant thereof, is the Methionine Adenosyltransferase 2A (MAT2A), preferably wherein said MAT2A has the sequence SEQ ID NO 5. The word "mutation" refers to a genetic modification including a modification that introduces a polynucleotide coding a polypeptide into a cell; a modification that substitutes, adds (inserts), or deletes one or more nucleotides of the genetic material of a parent cell; and chemical modification (exposure to a chemical) resulting in a change to the genetic material of a parent cell. Mutation includes a heterologous or homologous modification of referenced species. Mutation includes a modification of a coding region for polypeptide(s). Mutation also includes modification of non-coding regulatory regions that change expression of a gene or function of an operon. Non-coding regions include 5'-noncoding sequences (5' of a coding sequence) and 3'-noncoding sequences (3' of a coding sequence).

In a further embodiment of the present invention, in accordance with any one of the embodiments herein disclosed, said GAMT protein, a fragment or variant thereof, and said MAT protein, a fragment or variant thereof, are present in said cell in a ratio ranging from 10:1 to 1:10, preferably from 2:1 to 1:2. RBCs loaded with GAMT and MAT at the different ratios support the conclusion that a wide range of ratios could be used to metabolize GAA. The entire metabolic pathway from GAA to creatine is operative in RBCs loaded with recombinant human GAMT and MAT and methionine physiologically present in human plasma can sustain the formation of creatine.

In another embodiment, according to any one of the embodiments herein disclosed, the isolated cell modified for catalyzing the conversion of GAA into creatine in the presence of glucose and methionine using an exogenous guanidinoacetate methyltransferase (GAMT) and methionine adenosyltransferase (MAT) proteins, a fragment or variant thereof, is used as a medicament, in particular in the treatment of a subject suffering from a disease or condition caused by and/or characterized by a deficit of creatine.

In a further embodiment, in accordance with any one of the embodiments previously described, the isolated cell modified for catalyzing the conversion of GAA into creatine in the presence of glucose and methionine using an exogenous guanidinoacetate methyltransferase (GAMT) and methionine adenosyltransferase (MAT) proteins, a fragment or variant thereof, is used in the treatment of GAMT deficiency, creatine transporter deficiency (Dunbar M et al, 2014), methionine-dependent tumors (Cellarier E et al, 2003).

A further object of the present invention refers to a pharmaceutical composition comprising a plurality of modified cells, modified for catalyzing the conversion of GAA into creatine in the presence of glucose and methionine using an exogenous guanidinoacetate methyltransferase (GAMT) and methionine adenosyltransferase (MAT) proteins, a fragment or variant thereof, according to any one of the embodiments herein described, and one or more carriers, diluent and excipients.

The pharmaceutical composition described herein is a composition suitable for the administration of erythrocytes, therefore it is a composition for parenteral administration preferably in physiological solution, but also, for example, aqueous suspensions (also glucose) or formulated according to what is described in the prior art. By way of example and not limiting, water or buffers, integrated with preservatives, stabilizers, sugars and minerals, etc. can be used as pharmacologically acceptable excipients. This composition can also be in lyophilized form for storage and reconstituted in a suitable carrier before use.

Carriers are chemical or multi-chemical compounds that do not significantly alter or effect the active ingredients of the compositions. Examples include water, alcohols such as glycerol and polyethylene glycol, glycerin, oils, salts such as sodium, potassium, magnesium and ammonium, fatty acids, saccharides or polysaccharides.

Carriers may be single substances or chemical or physical combinations of these substances.

In another embodiment, in accordance with any one of the embodiments herein disclosed, the pharmaceutical composition has a pH between 7 and 8 and is isotonic.

In a preferred embodiment of the present invention, according to any one of the preceding embodiments, the pharmaceutical composition is given to the patients by endovenous administration in order to achieve a systemic effect. Other routes of administration such as intraperitoneal, intraarticular, subcutaneous or intramuscular administration are suitable.

Another object of this invention is the pharmaceutical composition, according to any one of the embodiments herein disclosed, which is used as a medicament, in particular in the treatment of a subject suffering from a disease or condition caused by and/or characterized by a deficit of creatine.

In another embodiment, in accordance with the previous embodiments, the pharmaceutical composition is used in the treatment of GAMT deficiency, creatine transporter deficiency, methionine-dependent tumors.

In a further embodiment of this invention, according to any one of the embodiments herein discussed, an effective dose of said pharmaceutical composition is administered to said subject from once a week to once a month. Expression "effective dose" refers to a dose of a drug that produces a biological response. The term effective dose is used when measurements are taken *in vivo.*

It forms part of the present invention also an *in vitro* method for the preparation of a modified cell, in accordance with any one of the previous embodiments, comprising a step of modifying a cell, whereby said cell is capable of catalyzing the conversion of guanidinoacetate acid (GAA) into creatine in the presence of glucose and methionine using an exogenous guanidinoacetate methyltransferase (GAMT) and methionine adenosyltransferase (MAT) proteins.

An embodiment of the present invention, according to any one of the embodiments herein disclosed, is said *in vitro* method for the preparation of a modified cell comprising the following steps:

  i. providing an isolated unmodified cell;
  ii. modifying said unmodified cell to yield a modified cell comprising the guanidinoacetate methyltransferase (GAMT) protein, a fragment or a variant thereof and the methionine adenosyltransferase (MAT) protein, a fragment or a variant thereof.

In one embodiment, step ii consists in loading said unmodified cell with the GAMT protein, a fragment or a variant thereof, and with the MAT protein, a fragment or a variant thereof; and/or transforming said unmodified cell with one or more exogenous nucleic acids comprising at least a nucleic acid sequence encoding said GAMT protein, a fragment or a variant thereof, and at least a nucleic acid sequence encoding said MAT protein, a fragment or a variant thereof. The loading step can be carried out according to standard procedures, preferably the process disclosed in US 10,849,858. This process has proven to be reproducible and reliable in encapsulating quantities of the substance of interest in red blood cells in proportion to the initial amount of said substance: these characteristics allow the clinical use of different doses, making it possible to administer doses commensurate to different clinical needs, varying only the amount of active ingredient added during the process. Any modification of the standard procedures is discussed in depth in the methods' section.

In a particularly preferred embodiment, according to any one of the previous embodiments, the unmodified cells are isolated from a subject suffering of Guanidinoacetate methyltransferase (GAMT) deficiency.

In another embodiment, in accordance with any one of the previous embodiments, the unmodified cells are isolated from a healthy subject. The protocol that has been followed for isolating the cells is described in the document ITRM2013061A1.

The invention encompasses also a medical treatment comprising the following steps:

  i. Isolate the unmodified cells from a subject suffering of Guanidinoacetate methyltransferase (GAMT) deficiency or a healthy subject;
  ii. Modify said unmodified cell to yield a modified cell comprising the guanidinoacetate methyltransferase (GAMT) protein, a fragment or a variant thereof and the methionine adenosyltransferase (MAT) protein, a fragment or a variant thereof.
  In particular, step ii consists in loading said unmodified cell with the GAMT protein, a fragment or a variant thereof, and with the MAT protein, a fragment or a variant thereof; and/or transforming said unmodified cell with one or more exogenous nucleic acids comprising at least a nucleic acid sequence encoding said GAMT protein, a fragment or a variant thereof, and at least a nucleic acid sequence encoding said MAT protein, a fragment or a variant thereof.
  iii. optionally prepare a pharmaceutical composition comprising a plurality of said modified cells and one or more carriers, diluent and excipients.
  iv. Administer said cells or pharmaceutical composition to the patient by endovenous administration in order to achieve a systemic effect.

Hence disclosed is a method for treating a subject suffering from a disease or condition caused by and/or characterized by a deficit of creatine.

In particular, it is herein disclosed the use of the pharmaceutical composition to treat GAMT deficiency, creatine transporter deficiency, methionine-dependent tumors.

In any part of the present description and claims the term comprising can be substituted by the term "consisting of".

Examples are reported below which have the purpose of better illustrating the methodologies disclosed in the present description, such examples are in no way to be considered as a limitation of the previous description and the subsequent claims.

## Examples

## Materials

The Change-IT TM Multiple Mutation Site Directed Mutagenesis Kit was obtained from Affymetrix. Mutagenic primers as well as sequencing primers, were synthesized by Sigma-Aldrich (Italy). The QIAprep® Miniprep Kit and the Plasmid midi-kit used for purification of plasmid DNA were from Qiagen. Tryptone, Yeast extract, Agar, Vegetable Peptone of microbiology grade as well as Ampicillin and all the chemical components of Synthetic Medium were from Sigma-Aldrich. IPTG was obtained from VWR International. The Ni-Sepharose™ High Performance resin used for Immobilized metal ion affinity chromatography (IMAC) was purchased from GE Healthcare Life Sciences. Centricon centrifugal filter devices, with Ultracel 10K membrane - 10,000 NMWL, used for recombinant enzyme concentration, were from Merck Millipore. Low molecular weight (LMW) Calibration Kit for SDS Electrophoresis was from GE Healthcare Amersham.

Sodium phosphate dibasic anhydrous ($Na_2HPO_4$), sodium dihydrogen phosphate ($NaH_2PO_4$) sodium dodecyl sulfate (SDS), phosphoric acid ($H_3PO_4$), acetonitrile (HPLC grade), Tris-HCl, dithiothreitol (DTT), ethylenediaminetetraacetic acid (EDTA), SAM, GAA, Cr, perchloric acid (PCA), sodium acetate, trichloroacetic acid (TCA) and octanesulfonic acid sodium salt, were obtained from Sigma-Aldrich. Polypro Hydrophilic Polypropylene membrane filters (13 mm, 0.2 $\mu$m) were obtained from Life Science (Ann Arbor, MI, USA). [Methyl-3H] methionine and scintillation fluid Emulsifier Scintillator Plus were obtained from PerkinElmer, Inc. (Boston, USA). The ion-exchange columns Discovery DSC-SCX SPE were purchased from Supelco Analytical (Sigma). All other chemicals used were obtained from Carlo Erba (Milan, Italy) and were of analytical grade.

## Methods

## Preparation of pET45b/His-UB-GAMT construct

Total RNA was extracted from the cervical cancer cell line HeLa by RNeasy Plus Mini Kit (Qiagen) and quantified by NanoDrop (Technologies, Wilmington, DE). RNA (1 $\mu$g) has been retrotranscribed using SuperScript First-Strand Synthesis System (Invitrogen, Carlsbad, CA, USA), oligo-dT (0.5 $\mu$g/reaction) and random hexamers (0.15 $\mu$g/reaction) in a final volume of 20 $\mu$l. Specific degenerate primers (reported in Table 1) were designed to obtain the PCR product of interest for the human GAMT CDS (NCBI Reference Sequence: NM_000156.5), which was initially cloned in the expression vector pET45b downstream of the His-tag coding sequence, using the PmlI and HindIII restriction sites. Next, the GAMT coding sequence was amplified, using a highly processive DNA polymerase with proofreading 3'-5' exonuclease activity, with a new forward primer (carrying a NcoI cutting site, shown in Table 1) and the same reverse primer used above and cloned in the pET45b-UB, an expression vector engineered to contain the ubiquitin coding sequence downstream of the His-tag. The final construct was referred to as pET45b/His-UB-GAMT. This strategy allows the production of GAMT protein, deleted of the initial L-Met ($\Delta$Met1) as occurs in the mature form of the human enzyme, directly fused at the C-terminus of the $(His)_6$-UB. The PCR product, purified by the QIAquick PCR Purification Kit (Qiagen) and quantified by NanoDrop, was digested with NcoI, blunted using Mung Bean Nuclease and then cut with HindIII before ligation with the pET45b-UB vector, linearized with Bam HI, subjected to Klenow fill in reaction, before digestion with HindIII and alkaline phosphatase treatment. The ligase reaction was used to transform the Novablue Escherichia coli (E. coli) strain following a standard protocol: incubation 30 min on ice; heat pulse for 90 s at 42°C and then 5 min on ice; addition of 200 $\mu$l SOC medium, followed by 1 h incubation at 37°C, before plating the transformation reaction on LB agar plates containing 100 $\mu$g/ml ampicillin. Positive colonies were screened by PCR using the primers T7 promoter and T7 terminator. Plasmid DNA was purified from different positive clones with the QIAprep Spin Miniprep Kit (Qiagen) and confirmed by sequencing. A positive clone was selected to be transformed into the BL21(DE3) expression host (as detailed below) to obtain the recombinant GAMT precursor, referred to as His-UB-GAMT. The NO-tag enzyme was obtained by digestion with the rHis-USP2 (Ubiquitin Specific Peptidase 2), a deubiquitylating enzyme which removes the His-UB from the fusion product (Figure 2A).

**Table 1. Primers used for GAMT cloning, site-directed mutagenesis and sequencing.**

| NAME | SEQUENCES (5' to 3') | NOTES |
|---|---|---|
| **Cloning primers** | | |
| GAMT PmlI_Fwd | ACGGTACTTCACGTGATGAGCGCCCCCAGCGCGA | |
| UB-GAMT NcoI_Fwd | ACGGTACTTCCATGGGCCCCCAGCGCGACCCCC | |
| GAMT HindIII_Rev | GGATACCTAAAGCTTCAGCCTTTGGTCACCAGGGGCG | |
| **Mutagenesis primers** | | **Codon change (aa change)** |
| UB-GAMT (L37I) | CACGCACCTGCGCATC**ATT**GGCAAGCCGGTGATGG | CTG → ATT (Leu→Ile) |
| UB-GAMT (G38A) | ACCTGCGCATCCTGG**C**CAAGCCGGTGATGGAGC | GGC → GCC (Gly→Ala) |
| UB-GAMT (L37I -G38A) | CACGCACCTGCGCATC**ATTGC**CAAGCCGGTGATGGAGC | CTG → ATT (Leu→Ile) GGC → GCC (Gly→Ala) |
| UB-GAMT (L37M) | ACGCACCTGCGCATC**A**TGGGCAAGCCGGTGATGG | CTG → ATG (Leu→Met) |
| UB-GAMT (I187Q) | GTACTCAGACATCACC**CAG**ATGTTTGAGGAGACGCAGGTG | ATC → CAG (Ile→Gln) |
| UB-GAMT (A26N) | GGGCGGCGCCCGCG**AA**CTACGACGCAGCGGAC | GCC → AAC (Ala→Asn) |
| UB-GAMT (V215Q) | GGAGGTGATGGCGCTG**CAG**CCACCGGCCGACTGCC | GTC → CAG (Val→Gln) |
| **Sequencing primers** | | |
| GAMT 7*_Fwd | GCCCCCAGCGCGACCCCCATCT | |
| GAMT 312*_Fwd | ACGGCAGACACACAAGGTCATCC | |
| GAMT 711*_Rev | TCAGCCTTTGGTCACCAGGGGC | |
| T7 promoter | TAATACGACTCACTATAGGG | |
| T7 terminator | GCTAGTTATTGCTCAGCGG | |

Fwd, forward; Rev, reverse. *Numbers indicate the 5' position of the sequencing primers, referring to the GAMT coding sequence (NCBI Reference Sequence: NM_000156.5). Nucleotide changes in the mutagenesis primer sequences are in bold and underlined and respective codons are highlighted in grey. Codon changes and relative amino acid substitutions are reported in the third column. Restriction enzyme cutting sites in cloning primers are underlined.

**Site-directed mutagenesis of pET45b/His-UB-GAMT construct**

Mutagenesis was carried out by using the Change-IT TM Multiple Mutation Site Directed Mutagenesis Kit, following the manufacturer's instructions, with slight modifications. All the reactions were performed using the wild-type pET45b/His-UB-GAMT expression construct as template and the mutagenic primers designed, according to the guidelines provided in the kit protocol, to introduce the targeted amino acid substitutions. Primer sequences are reported in Table 1. Briefly, a typical reaction was assembled, adding 1X Change-ITTM Buffer, 0.25 μM GAMT mutagenic primer(s), 0.25 μM AMP FWD primer (which anneals to the opposite plasmid strand respect to the mutagenic primer), 7.5 ng of plasmid DNA template and 0.4 μl of Change-IT Enzyme, in a final volume of 10 μl. The reaction was performed in the PCR 2700 Thermal Cycler. A 5 μl aliquot of the completed mutagenesis reaction has been removed to a fresh tube, added with 0.5 μl DpnI and incubated at 37°C for 2 h.

**Selection of mutagenized clones**

E. coli Novablue competent cells (50 μl) were transformed with the DpnI-treated mutagenesis reaction (3 μl) following the standard heat-shock protocol detailed above. Seven clones were amplified for plasmid purification performed by QIAprep Spin Miniprep Kit, according to the manufacturer's instructions. The successful of the mutagenesis reactions was confirmed by DNA sequencing of the expression constructs, using the dideoxy-termination method and a capillary sequencer (PE 310 Perkin Elmer). Sequencing primers are reported in Table 1. The selected clones bearing the desired mutagenesis, were amplified in Novablue for midi-scale plasmid DNA recovery: 100 ml culture volume was processed for large scale plasmid DNA extraction following the Qiagen Plasmid Midi Kit protocol. Plasmid DNA, resuspended in TE buffer, was stored at -20°C.

**Establishment of GAMT expressing BL21(DE3) E. coli strain**

BL21(DE3) competent cells (50 μl) were transformed with 1 μl of a 2ng/μl dilution of the different mutagenized GAMT expression vectors, following the transformation protocol detailed above for Novablue. A few clones of BL21(DE3), bearing the different pET45b/His-UB-GAMT expression constructs, were tested for GAMT expression.

**Establishment of Bacterial Glycerol Stocks for Long-term Storage of Plasmids, from both Novablue and BL21(DE3) strains**

A single colony was picked from a freshly streaked VP + ampicillin (100 μg/ml) plate. The bacterial culture was incubated at 37°C with shaking at 250 rpm, until optical density at 600 nm (O.D.600) reached approximately 0.7-0.8 (4-6 h required). Then, 900 μl of the bacterial culture were added to 600 μl of glycerol, previously dispensed in the cryovials. One hundred and fifty glycerol stock tubes of E. coli cells (Novablue and BL21(DE3), transformed with each GAMT construct, identified as Research Cell Bank (RCB) were prepared and immediately frozen at -80°C.

**Induction of rhGAMT at lab-scale level**

The "standard small-scale" induction protocol is described below (any variation of induction parameters is reported in the "Results" section). Starting culture: an isolated colony was picked from a freshly streaked plate and grown overnight (ON) at 37°C in 10 ml LB + ampicillin (100 μg/ml) in a 50 ml tube on a benchtop orbital shaker. A sample of the overnight culture was withdrawn and diluted 1:10 before reading O.D.600, in a spectrophotometer. The ON inoculum was then diluted into 100 ml LB + ampicillin (100 μg/ml) in order to reach an O.D.600 of about 0.1 and put in a 0.5 liter flask. The culture was then incubated at 37°C with aeration and shaking at 250 rpm. Bacterial growth was monitored, at regular intervals, until culture density reached an O.D.600 of about 0.5-0.6. 10 ml of culture were then removed and transferred to a labeled 50 ml tube and let grow at 37°C (this is the Not Induced control, NI). IPTG at 1 mM final concentration was added into the flask to induce protein production (this is the Induced sample, I). At 1 h-intervals, up to 4 h, 10 ml of induced bacterial culture were withdrawn, transferred into a 15 ml-tube and put on ice. Cells were collected by centrifugation at 2750 g for 20 min at 4°C, the supernatant was removed and the pellet frozen at -20°C for later processing.

**Preparation of bacterial extracts and SDS-PAGE analysis**

Cell lysis was obtained by resuspension of the cell pellets (from both NI and I sample) in 0.05 culture volume of ice-cold Lysis Buffer (20 mM Na/K Phosphate buffer pH 7.5, 15 mM β-Mercaptoethanol, 15% (v/v) glycerol, 500 mM NaCl), followed by three cycles (30 s each) of sonication at 75 watts, while keeping the sample on ice. Samples were centrifuged 10 min at 9600 g at 4°C: the supernatant (S), corresponding to the soluble cytoplasmic fraction, was transferred into a new microcentrifuge tube, while the residual pellet was resuspended in Lysis Buffer and sonicated as above; this further sample has been referred to as the Pellet (P) sample.

The protein concentration in both S and P samples was determined by the Bradford assay (Bradford, 1976), to normalize the samples for loading.

For SDS-PAGE, an aliquot corresponding to 20 $\mu$g of proteins (for P loaded 10$\mu$l), was combined with an equal volume of 2X Sample Buffer (2X SB = 2% SDS, 100 mM Tris-HCl, pH 6.8, 20% glycerol, 0.0025% w/v bromophenol blue) and boiled 1 min at 100°C to denature proteins. Proteins were resolved by 12% (w/v) polyacrylamide gel electrophoresis, in parallel with the Low Molecular Weight standard (LMW) as a size reference.

## Immobilized metal ion affinity chromatography (IMAC) for His-UB-GAMT purification

The recombinant His-tagged UB-GAMT in the supernatant fraction, was purified by the immobilized metal ion affinity chromatography (IMAC), exploiting the interaction between chelated transition metal ions (Ni$^{2+}$) and side-chains of histidines (His) on proteins, essentially according to the manufacturer's instructions. Briefly, the column was equilibrated with the Binding buffer which corresponds to the Lysis Buffer in which the sample is resuspended. After sample loading, washings were initially performed with the same Binding buffer, and then with Binding Buffer containing 40 mM imidazole. Elution of the His-tagged recombinant protein was obtained by Binding buffer containing 350 mM imidazole.

## USP2 digestion of His-UB-GAMT and GAMT purification

His-UB-GAMT obtained upon Ni-Sepharose affinity chromatography was digested with the recombinant His-tagged deubiquitinating enzyme USP2 (rHis-USP2) to remove the His-UB partner fused at the N-terminus of GAMT. For optimal digestion, His-UB-GAMT and rHis-USP2 were combined allowing a mass ratio in the range of 200:1 and 100:1 and incubated at least 3 h at 37°C, in the same GAMT Elution buffer, suitably diluted by loading buffer in order to keep the imidazole concentration $\leq$ 50 mM.

GAMT NO-tag was purified from all the His-tag byproducts generated during digestion, not-digested His-UB-GAMT and His-USP2, by Nickel affinity chromatography. Recovery yield was calculated upon the protein concentration assay with Bradford (Bradford, 1976), while the purity of the enzyme was assessed by SDS-PAGE. GAMT was stored at -80°C (in the same binding buffer).

## Determination of GAMT activity

The evaluation of enzyme activity was performed by a method based on the quantification of creatine. The dosage method was carried on according to Alessandri MG et al, 2004 with some modifications. Briefly, in a final volume of 250 $\mu$l, the reaction mixture contains 50 mM Tris-HCl (pH 7.5), 2 mM DTT, 0.25 mM SAM, 1 mM GAA, and GAMT (in our experiment it has been standardized on amount of 10 $\mu$g). The incubation was carried out at 37°C for 15 min and at different incubation times (0-5-10-15 min) the reaction was stopped by the addition of 250 $\mu$l of 1 N PCA. The reaction mixture was then centrifuged at 18,500 g for 15 min at 4°C, and 50 $\mu$l of the clear supernatant was injected into the HPLC after filtration with 0.2 $\mu$m filter into special vials. Blanks, containing the reaction mixture without enzyme, were analyzed as well. Values are expressed as $\mu$mol creatine/min/mg protein.

Chromatographic conditions

The analysis was performed by the method of reverse phase chromatography at 30°C and using a gradient elution system as reported in Table 2 A. The mobile phase consisted of buffer A: Na$_2$HPO$_4$ (7.1g), SDS (2g), distilled water (750 ml) and buffer B: Na$_2$HPO$_4$ (7.1g), SDS (2g), distilled water (500 ml), acetonitrile (250 ml). Both buffers reached a final pH 3 by H$_3$PO$_4$. Before use, the buffers were filtered by Polypro filters. The increase of creatine levels has been observed after different analysis times (0-15 min).

**Table 2: Gradient elution system used in HPLC detection of GAMT (A) and MAT (B) activity.**

| A. GAMT CHROMATOGRAPHIC METHOD | | | B. MAT CHROMATOGRAPHIC METHOD | | |
|---|---|---|---|---|---|
| Time (min) | % buffer A | % buffer B | Time (min) | % buffer A | % buffer B |
| 0-2 | 100 | 0 | 0-8 | 80 | 20 |
| 2-20 | 0 | 100 | 8-8.5 | 60 | 40 |
| 20-22 | 0 | 100 | 8.5-21 | 60 | 40 |

(continued)

| A. GAMT CHROMATOGRAPHIC METHOD | | | B. MAT CHROMATOGRAPHIC METHOD | | |
|---|---|---|---|---|---|
| Time (min) | % buffer A | % buffer B | Time (min) | % buffer A | % buffer B |
| 22-25 | 100 | 0 | 21-21.5 | 80 | 20 |
| 25-30 | 100 | 0 | 21.5-31.5 | 80 | 20 |

**GAA uptake by RBCs**

The ability of GAA to enter into RBCs has been evaluated. In detail, RBCs at 7.5 % Ht were incubated for 1 h at 37°C in the presence of different $^{13}C_2$ GAA (GAA*) amounts (0-100 $\mu$M). At different incubation times (0, 5, 10, 15, 30 and 60 min), 600 $\mu$l-aliquots were stratified onto 600 $\mu$l 1-Bromododecane and centrifuged 3 min at 7,840 g to obtain a better separation between RBCs and $^{13}C_2$ GAA. Supernatants were discarded, RBCs recovered in a physiological saline solution containing: 10 mM 2-[4-(2-hydroxyethyl)piperazin-1-yl]-ethanesulfonic acid (HEPES, pH 7.4), 154 mM NaCl and 5 mM glucose (Hepes solution) to a final 50% Ht. GAA* content was analyzed on dried blood spots (dbs) by Tandem mass spectrometry as described by Carducci C et al, 2006.

**Loading of GAMT into human RBCs**

Whole blood (WB) was obtained from healthy volunteers included in the Italian blood donor registry (registered A.V.I.S. donors) who signed an informed consent form. Blood was collected in heparinized tubes and provided by the "Santa Maria della Misericordia" Hospital in Urbino. Briefly, GAMT was loaded into human RBCs by means of hypotonic dialysis, isotonic resealing and re-annealing, according to Magnani M et al, 1988 with some modifications.

Whole blood was centrifuged 10 min at 4°C at 1,800 g to remove plasma and then washed twice by 10 min centrifugation at 4°C, at 1,800 g, in Hepes solution. The dialysis procedure was carried out with the addition of 220 $\mu$l of protein solution (corresponding to 340 $\mu$g GAMT in a 1.56 mg/ml solution) to RBCs suspended in Hepes solution at 70% Ht, in a final volume of 1 ml. The suspension was dialyzed for 90 min at 4°C in a cellulose tube (14 kDa MWCO, Roth, Karlsruhe, Germany), placed in a rotating plate versus 50 ml of hypotonic dialysis buffer optimized for human RBC loading containing 10 mM $NaH_2PO_4$, 10 mM $NaHCO_3$ (pH 7.4), 20 mM glucose, 3 mM GSH and 2 mM ATP. The final osmolarity of the hypotonic solution was 72 mOsm, as measured by the Osmometer Fiske Associates, Model 210 (Norwood, MA, USA). After dialysis, cells reached about 123 mOsm. Subsequent resealing and re-annealing steps were carried out by incubating the pooled dialyzed RBC suspensions 5 min at 37°C. Then, PIGPA solution (100 mM inosine, 20 mM ATP, 10 mM anhydrous glucose, 100 mM sodium pyruvate, 4 mM $MgCl_2$, 190 mM NaCl, 1666 mM KCl and 33 mM $NaH_2PO_4$) was added to the RBCs (10% v/v) to restore isotonicity (approx. 300 mOsm) and the suspension was incubated 25 min at 37°C under gentle stirring, to allow pore closure. The final washing phase in physiological saline solution was carried out twice, centrifuging 10 min at 500 g and 4°C. Final packed loaded RBCs were re-suspended in Hepes solution pH 7.4, at about 40% Ht and the amount of entrapped GAMT evaluated on RBC lysates following the method mentioned above. As a result, we obtained human RBCs loaded with 0.01 IU/ml cells at 100% Ht, corresponding to 0.138 mg of GAMT per ml cells at 100% Ht. By increasing the amount of protein added during the dialysis step (660 $\mu$g) and by replacing the dialysis buffer after 45 min with a fresh one (reaching RBCs 97 mOsm), cells loaded with 0.043 IU/ml RBCs at 100% Ht, corresponding to 0.53 mg/ml RBCs, were obtained.

**Evaluation of RBC SAM levels**

Extraction procedure

SAM was extracted as reported in Wise CK et al, 1997. Briefly, frozen packed RBCs were added with an equal volume of ice-cold 0.1 M sodium acetate, pH 6 and vortex-mixed. The protein was precipitated by adding 40% (w/v) TCA equal to one-fifth of the original volume of the RBC solution and placed on ice for 30 min to complete precipitation. To remove precipitated protein the tubes were centrifuged at 25,000 g for 10 min at 4°C. The supernatant containing SAM was added with an equal volume of ice-cold peroxide-free diethyl ether to extract lipids (ether extraction step). The tubes were vortex-mixed for 20 s and centrifuged to separate the phases. The top layer was drawn off and discarded. The ether extraction step was repeated once. The samples were filtered and injected into HPLC system.

Chromatographic conditions

The assay has been performed as reported in Wang W et al, 2001. Briefly, the mobile phase consisted of two solvents: Solvent A, 8 mM octanesulfonic acid sodium salt and 50 mM $NaH_2PO_4$ adjusted to pH 3.0 with $H_3PO_4$ and Solvent B, 100% methanol. The HPLC column was equilibrated with 80% Solvent A and 20% Solvent B. The sample was injected and separation was obtained using a step gradient. The gradient consisted of 8 min at the equilibration conditions, 30 s to increase Solvent B to 40%, 12.5 min at the new condition, and 30 s to return to the equilibration conditions and a minimum of 10 min before a subsequent injection. The flow-rate was 1 ml/min and detection was monitored at 254 nm. SAM standard was dissolved in water at a concentration of 1 mM and then diluted to 0.05 mM with 0.4 M PCA.

**Determination of RBC MAT activity**

Preparation of RBC lysate

RBCs were washed three times in 10 volumes of 5 mM sodium phosphate, 150 mM NaCl, pH 7.4 and centrifuged at 1,800 g for 10 min at 4°C. The supernatant was aspirated and final RBC pellet was lysed in 4 volumes of ice-cold deionized water and incubated for 20 min on ice. The membrane fraction was pelleted by centrifugation at 20,000 g for 1 h and cytosolic fraction was recovered from the supernatant. The cytosol was dialyzed overnight against 30 mM KCl, 40 mM Hepes pH 7.4 at 4°C.

Assay of MAT catalytic activity

MAT activity was assayed by measuring the rate of formation of [3H]SAM from ATP and L-[methyl-3H] methionine as reported in Cheng H et al., 1997 with some modifications. Briefly, the reaction mixture (total volume of 0.1 ml, pH 7.4) contained 50 $\mu$l of cell lysate and final concentrations of 30 mM $MgCl_2$, 26 mM KCl, 35 mM Hepes, 10 mM ATP and 20 $\mu$M L-[methyl-3H]methionine (12,600 cpm/nmol). The samples were incubated at 37°C for 60 min and the reaction was stopped by the addition of 1.0 ml ice-cold 1.6 mM citric acid in 50% ethanol. The time 0 of reaction was used as blank. After adsorption to an ion-exchange resin (column in the $NH_4^+$ form), S-adenosyl-[methyl-3H]methionine was eluted with 1.0 ml of 3.0 M $NH_4OH$ into a vial containing 5 ml of scintillation fluid. The samples were counted in a Packard scintillation counter. The results of MAT activity were expressed as pmol/mg/min.

Assay of protein concentration

Protein concentration was estimated by the Lowry procedure (Bailey JL, 1962).

**Generation of pET45b/His-UB-MAT2A construct**

The MAT coding sequence of the human MAT2A gene (DQ083239.1) was amplified from cDNA obtained from HeLa cells (see GAMT cloning strategy), with specific degenerate primers designed to provide the restriction sites (SacII and NotI) useful for cloning. Primer sequences are reported in Table 3. The MAT2A PCR product was cloned downstream of $(His)_6$-ubiquitin coding sequence into the pET45b-UB expression vector.

The PCR product, purified by the QIAquick PCR Purification Kit (Qiagen) and quantified by NanoDrop, was cloned in the pET45b-UB vector by using the NotI/SacII restriction enzymes. The MAT coding PCR product was thus inserted downstream of $(His)_6$-ubiquitin coding sequence into the pET45b-UB expression vector. The ligase reaction was used to transform the Novablue E. coli strain following the standard heat-pulse protocol: incubation 30 min on ice; heat pulse for 90 s at 42°C and then 5 min on ice; addition of 200 $\mu$l SOC medium, followed by 1 h incubation at 37°C, before plating the transformation reaction on LB agar plates containing 100 $\mu$g/ml ampicillin. Positive colonies were screened by PCR. Plasmid DNA of PCR-positive clones was purified with the QIAprep Spin Miniprep Kit (Qiagen) and confirmed by sequencing. Primer sequences are reported in Table 3. A positive clone was chosen to obtain the expression strain.

**Table 3. Primers used for MAT cloning and sequencing.**

| NAME | SEQUENCES (5' to 3') | NOTES |
|---|---|---|
| **Cloning primers** | | |
| MAT2A SacII_Fwd | CGTCT<u>CCGCGG</u>TGGAATGAACGGACAGCT CAACGGC | |
| MAT2A NotI_Rev | CGTCT<u>GCGGCCGC</u>TCAATATTTAAGCTTTT TGGGCACTTCCC | |

(continued)

| Sequencing primers | | |
|---|---|---|
| MAT2A 100*_Fwd | GTGACCAAATCAGTGATGCTGTCC | |
| MAT2A 398*_Fwd | AGACCAGGGCTTAATGTTTGGC | |
| MAT2A 799*_Fwd | TTGTGGACACTTATGGCGGTTG | |

Fwd, forward. Rev, reverse *Numbers indicate the 5' position of the sequencing primers, referring to the MAT coding sequence (NCBI Reference Sequence: DQ083239.1). Restriction enzyme cutting sites in cloning primers are underlined.

The following steps were performed exactly as detailed for His-UB-GAMT:

- Establishment of MAT expressing BL21(DE3) E. coli strain

- Establishment of Bacterial Glycerol Stocks for Long-term Storage of Plasmids, from both Novablue and BL21(DE3) strains.

- Expression studies performed at lab-scale (Induction of recombinant MAT2A)

- Preparation of bacterial extracts and SDS-PAGE analysis

**Immobilized metal ion affinity chromatography (IMAC) for His-UB-MAT purification**

MAT purification was carried out by IMAC as described for His-UB-GAMT, the only exception being the composition of the lysis buffer: (40 mM Na/K Tris-HCl, 2.2 mM KCl, 20% (v/v) glycerol, 0.04% Tween20, 110 mM NaCl).

**USP2 digestion of His-UB-MAT**

The same protocol of USP2 digestion used for rHis-UB-GAMT was followed.

**Ion-exchange chromatography by Diethylaminoethyl (DEAE) for MAT**

NO-tag MAT was purified from all the His-tag byproducts generated during the digestion and His-USP2 itself, by DEAE chromatography. Briefly, the column was equilibrated with the binding buffer (40 mM Na/K Tris-HCl, 2.2 mM KCl, 20% (v/v) glycerol, 0.04% Tween20, pH 8.0). After sample loading, washings were initially performed with the same binding buffer, and then with binding Buffer containing 50 mM NaCl. Elution of the recombinant protein was obtained by binding buffer containing 300 mM NaCl. The NaCl concentration was then brought back to 110 mM. Before scaling-up, the lysis process and the binding buffer were optimized thus leading to increased protein activity. Recovery yield was calculated by Bradford MM (1976) while the purity of the enzyme was assessed by SDS-PAGE. MAT was finally stored at -80°C. In Figure 3 the expression strategy to obtain NO-tag MAT is summarized.

**Determination of MAT activity**

The evaluation of enzyme activity was performed by a method based on the quantification of S-(5'- Adenosyl)-L-methionine chloride dihydrochloride (SAM). The dosage method was performed according to Shiozaki S et al, 1984 with some modifications. Briefly, in a final volume of 250 $\mu$l, the reaction mixture contained 100 mM Tris-HCl (pH 7.5), 50 mM $MgCl_2$, 100 mM KCl, 8 mM GSH, 20 mM ATP, 20 mM L-Met and MAT (in our experiment it has been standardized on amounts of 10 and 20 $\mu$g). The incubation was carried out at 37°C for 10 min: at different incubation times (0-1-2.5-5-10 min) the reaction was stopped by the addition of 250 $\mu$l of 2 N PCA. After filtration with 0.2 $\mu$m filter, 50 $\mu$l of reaction mixture were injected into the HPLC. Blanks, containing the reaction mixture without enzyme, were analyzed as well.

Chromatographic conditions

The analysis was performed as reported by Wang W et al, 2001 with some modifications: the method was a reverse phase chromatography and the detection was monitored at 254 nm, at 25°C using a gradient elution system at a flow-rate of 1 ml/min as reported in Table 2 B. The mobile phase consisted of buffer A (8 mM $C_8H_{17}O_3SNa$ and 50 mM $NaH_2PO_4$) and buffer B ($CH_3OH$ 100%. Buffer A reached a final pH 3 by $H_3PO_4$) and it was filtered with Polypro filters before use. Before and after sample analysis, the column was equilibrated and standard solution of 0.05 mM of SAM in 1 N HClO$_4$ was analyzed. SAM peak was identified according to its retention time and co-chromatography with SAM standard. The increase of SAM levels after different analysis times (0-10 min) has been observed. Quantification was based on integration of peak areas and compared to the standard calibration curves of SAM.

**Co-entrapment of GAMT and MAT into human RBCs**

Whole blood (WB) was obtained from healthy volunteers included in the Italian blood donor registry (registered A.V.I.S. donors) who signed an informed consent form. Blood was collected in heparinized tubes and provided by the "Santa Maria della Misericordia" Hospital in Urbino. Briefly, GAMT/MAT were loaded into human RBCs by means of hypotonic dialysis, isotonic resealing and re-annealing, according to Magnani M et al (1988) with some modifications.

Whole blood was centrifuged 10 min at 4°C at 1,800 g to remove plasma and then washed twice in Hepes solution by 10 min centrifugation at 1,800g at 4°C The dialysis procedure was carried out with different concentrations of proteins added to RBCs suspended in Hepes solution at 60% Ht, as follows:

A. 125 μg (0.025 IU) of enzyme GAMT (50 μl protein solution [2.5 mg/ml] with SA 0.2 IU/mg);

B. 125 μg (0.025 IU) of enzyme MAT (125 μl protein solution [1 mg/ml] with SA 0.2 IU/mg);

C. 125 μg (0.025 IU) of enzyme GAMT (50 μl protein solution [2.5 mg/ml] with SA 0.2 IU/mg)

and 125 μg (0.025 IU) of enzyme MAT (125 μl protein solution [1 mg/ml] with SA 0.2 IU/mg);

D. 125 μg (0.025 IU) of enzyme GAMT (50 μl protein solution [2.5 mg/ml] with SA 0.2 IU/mg)

and 250 μg (0.050 IU) of enzyme MAT (250 μl protein solution [1 mg/ml] with SA 0.2 IU/mg);

E. 250 μg (0.050 IU) of enzyme GAMT (100 μl protein solution [2.5 mg/ml] with SA 0.2 IU/mg)

and 125 μg (0.025 IU) of enzyme MAT (125 μl protein solution [1 mg/ml] with SA 0.2 IU/mg).

All the conditions (in 1 ml final volume) were dialyzed for 90 min at 4°C in a cellulose tube (14 kDa MWCO, Roth, Karlsruhe, Germany), placed in a rotating plate versus 50 ml of the hypotonic dialysis buffer previously described replacing the buffer with fresh one after 45 min. The final osmolarity of the hypotonic solution was 68±2 mOsm, as measured by Osmometer Fiske Associates, Model 210 (Norwood, MA, USA). After dialysis, the cells reached about 90±4 mOsm.

Subsequent resealing and re-annealing steps were carried out by incubating the pooled dialyzed RBC suspensions 5 min at 37°C. Then, PIGPA was added to the RBCs (10% v/v) to restore isotonicity (approx. 300 mOsm) and the suspensions were incubated 25 min at 37°C under gentle stirring, to allow pore closure. The final washing phase in physiological saline solution was carried out twice, centrifuging 10 min at 500 g and 4°C. Unloaded RBCs submitted to the same procedure without the addition of the recombinant proteins were used as controls. Final packed loaded RBCs were re-suspended in phosphate buffered saline solution (PBS) pH 7.4, at about 40% Ht in presence of 200 μM $^{13}$C$_2$ GAA and 200 μM L-Met and incubation of these suspensions (A-E) were performed for 5 hours at 37°C. At the planned time points (0-1-2-3-4-5h) 50 μl-aliquot of each suspension was collected on a standardized filter paper and the dbs obtained were submitted to biochemical monitoring of $^{13}$C$_2$ GAA, $^{13}$C$_2$ Cr and L-Met by MS/MS analysis by Department of Molecular Medicine, La Sapienza University of Rome (Carducci C et al, 2006).

**Results**

**Example 1 - Cloning strategies for the production of recombinant human GAMT**

Different expression strategies were explored to obtain a recombinant human GAMT, with the final aim to produce an enzyme useful for therapeutic purposes.

In a first attempt, the GAMT coding sequence was cloned in a pET45b expression plasmid directly fused to the His-tag coding sequence. The expression vector has been transformed in the BL21(DE3) E. coli strain and the clone showed a good expression of the N-terminal His-tagged recombinant protein, although the most part was in the insoluble fraction. Different induction protocols were tested, changing the temperature and/or the length of induction, the cell density at which GAMT expression was induced, but in no case any improvement was observed.

To overcome the drawbacks of the His-tag fusion strategy and to finalize the expression for therapeutic use, we designed and prepared two further constructs, using both pET45b and pET22b expression vectors in which the GAMT insert was cloned in such a way to produce the amino acid sequence of GAMT without any extra part (NO-tag GAMT).

Unfortunately, the GAMT protein was expressed in a very low amount (or not expressed at all) and this result was not overcome by using different experimental conditions (different expression host, use of synthetic media). Moreover, a low growth rate was observed upon IPTG induction, which opened the hypothesis that GAMT might be toxic for bacteria, considering that it uses as substrate SAM that is very important in different endogenous pathways, like bacterial cell division (Newman et al., 1998).

Following the hypothesis of GAMT toxicity for the host expressing cells, a construct referred to as pET22b-pelB-GAMT was generated, to target GAMT into the periplasmic space. The induction tests revealed that GAMT was expressed in the insoluble fraction as inclusion bodies. Unfortunately, protein purification from the insoluble fraction and refolding requires too much time, difficult approaches and, moreover, the strategy used does not ensures that the pelB signal is completely removed.

Then, a different expression strategy was attempted by cloning the GAMT CDS in the pET22b backbone in frame with the C-terminal His-tag provided in the cloning/expression region of the vector. However, this further expression construct (pET22b-GAMT-His-Tag) behaved like the pET22b-GAMT and pET45b-GAMT described above, with an undetectable recombinant GAMT expression. Thus, unexpectedly, only the N-terminal Tag-based strategies allowed the expression of the recombinant enzyme. Starting from this observation, a construct was designed and developed aimed both at expressing a N-terminal tagged GAMT precursor and at introducing a cleavage site to specifically remove the added tag. We have cloned GAMT in the pET45b-UB plasmid. Indeed, the recombinant His-UB-GAMT product can be treated with a deubiquitylating enzyme to remove the His-ubiquitin partner and release the natural GAMT protein. This construct, named pET45b/His-UB-GAMT, allowed the expression of the protein at high levels, although mostly partitioned in the insoluble fraction; furthermore, the purified enzyme showed low stability.

To address both solubility and stability issues, this construct underwent further optimization steps before project scaling-up.

## Example 2 - Kinetic activity and stability of native GAMT

The recombinant human GAMT-NO-tag showed a good affinity for its substrate GAA with Km value of 0.057 $\pm$ 0.009 mM and a specific activity (S.A.) of 0.08 $\pm$ 0.02 $\mu$mol/min/mg. However, most of the recombinant product was partitioned in the insoluble fraction (referred to as Pellet sample) and this raised a solubility issue (Figure 2 B). In addition, the enzyme did not show acceptable stability when stored at 4°C as reported in Table 4, where S.A. values of native GAMT, stored for different times (3 days and 1, 2, 3, 5 and 7 weeks) and at different temperatures (4°C, -20°C and -80°C) are summarized. Indeed, data show a rapid decrease of enzymatic activity already after 3 days at 4°C (0.05 $\pm$ 0.009 versus 0.08 $\pm$ 0.02 $\mu$mol/min/mg at time 0) until to a complete disappearance after 2 weeks of storage. This decrease is confirmed by the SDS-PAGE (Figure 2 C), in which an evident thinning of the band relating to the intact enzyme can be observed. Aliquots stored at -20°C and -80°C have unchanged values of S.A, highlighting the effectiveness of the conservation process, confirming what observed by SDS-PAGE.

**Table 4. Stability results of native GAMT at different temperatures and for different times.**

| Sample | S.A. 3 days | S.A. 1 week | S.A. 2 weeks | S.A. 3 weeks | S.A. 5 weeks | S.A. 7 weeks |
|---|---|---|---|---|---|---|
| **GAMT 4°C** | 0,050 $\pm$ 0,009 | 0,015 $\pm$ 0,002 | 0 | 0 | 0 | 0 |
| **GAMT - 20°C** | 0,095 $\pm$ 0,023 | 0,081 $\pm$ 0,003 | 0,080 $\pm$ 0,003 | 0,070 $\pm$ 0,005 | 0,098 $\pm$ 0,003 | 0,098 $\pm$ 0,002 |
| **GAMT - 80°C** | 0,088 $\pm$ 0,001 | 0,084 $\pm$ 0,006 | 0,078 $\pm$ 0,003 | 0,088 $\pm$ 0,002 | 0,098 $\pm$ 0,005 | 0,100 $\pm$ 0,001 |
| S.A. = Specific activity ($\mu$mol/min/mg) | | | | | | |

## Example 3 - Native GAMT engineering to increase protein stability and solubility

Preliminary in silico studies were performed, using different bioinformatic tools, to identify amino acid residues involved in GAMT stability. Based on evidences reported by Komoto J et al, 2002 for rGAMT from rat liver, we planned different site-directed mutagenesis to generate three mutagenized expression constructs, carrying the following amino acid substitutions: Leu37Ile (L37I); Gly38Ala (G38A); Leu37Ile and Gly38Ala (L37I-G38A) (Figure 4A). All the mutants proved to be stable under different conditions (stability of the double mutant GAMT L37I-G38A is shown in Figure 4B); however, the NO-tag single mutant L37I exhibited a lower specific activity respect to the wild-type enzyme (0.03 vs 0.08 nmol/min/$\mu$g) while for the mutant carrying the two amino acid changes (L37I-G38A), no activity was detected (Figure 4C). BLAST alignments run to identify highly conserved amino acids at the mutagenized positions, showed that the mutagenized residues (L37 and G38) are indeed highly conserved in all the GAMT proteins sequenced, the only exception being the replacement of Leu37 with Met in the Coturnix japonica (predicted sequence), Pundamilia nyererei (predicted sequence) and Branchiostoma floridae (predicted sequence). Therefore, in the next optimization studies, the Leu37Met (L37M) substitution was tested, instead of L37I, for its ability to preserve GAMT stability. Furthermore, the wild-type GAMT protein is prone to precipitation and indeed the induced recombinant enzyme is mainly found in the insoluble fraction. To address the solubility issue, further in silico studies were performed, using the different bioinformatic tools Yasara, TANGO, Aggrescan and Eris, to highlight the main intermolecular interactions, also based on the crystallographic structure of GAMT. Each amino acid change was selected considering: residues involved in catalytic-site formation; residues highly conserved; residues that may influence protein folding as well as protein stability and solubility. Bioinformatic solubility studies with Yasara (http://www.yasara.org/biotools.htm) and TANGO (http://www.switchlab. org/bioinformatics/tango) lead to the identification of Ile185 and Ile187 as residues with a high-aggregation potential. Ala26, situated in the N-terminal hydrophobic patch of GAMT, was predicted to mediate hydrophobic interactions between two GAMT monomers. Considering the free energy change ($\Delta\Delta G$) caused by the single point mutations (calculated by Yasara algorithm) and their influence on the intermolecular energy interactions (calculated by FoldX), the best candidates were: A26N; L37M; I187Q; V215Q (Figure 5). The predicted free energy change ($\Delta\Delta G$) of protein folding (based on Eris platform dynamic models) after the multi-site mutagenesis planned is reported below:

$$\Delta\Delta G \ (A26N - L37M - I187Q - V215Q) = -\ 1.93\ Kcal/mol.$$

Several mutagenesis were performed and three candidate clones, bearing different combinations of mutagenized residues, were selected for further studies:

<div align="center">

CL32: A26N-L37M-I187Q-V215Q

CL51: L37M-I187Q-V215Q

CL76: A26N-L37I-I187Q-V215Q

</div>

The small-scale induction of the mutagenized constructs, performed as described under "Methods", showed an improvement in terms of solubility for the three clones (CL32; CL51; CL76), with an increased amount of His-UB-GAMT in the soluble fraction, although most still remained in the pellet fraction. All GAMT mutants were stable, at the protein level, when maintained up to 42 h at 37°C, both in the His-UB-tagged form and after USP2-mediated His-UB removal (Figure 6). From now on, the term GAMT will refer to mutated forms.

## Example 4 - Activity and stability of three GAMT mutants

The enzymatic activity of CL32, CL51 and CL76 evaluated at the end of purification (time 0) were 0.15, 0.16, 0.09 U/mg protein, respectively. The analysis of GAMT stability showed that CL32 and CL51 maintained about 60 and 56% of activity after three days at 37°C, respectively, while CL76 exhibited about 33% activity under the same conditions, as shown in Table 5. The CL32 mutant has been selected as lead candidate for the subsequent studies (lab-scale expression and then scaling-up production by synthetic media).

<div align="center">

**Table 5: Stability results of the three GAMT mutants.**

| Stability at 37°C (nmol/min/$\mu$g) | 0h | 24h | 48h | 72h |
|---|---|---|---|---|
| **GAMT CL32** | 0.15 | 0.13 | 0.11 | 0.09 |
| **GAMT CL51** | 0.16 | 0.12 | 0.11 | 0.09 |
| **GAMT CL76** | 0.09 | 0.08 | 0.06 | 0.03 |

</div>

## Example 5 - Expression optimization of GAMT CL32 in BL21(DE3)

At first, expression studies were performed at lab-scale in LB medium; subsequently, to move towards a product for clinical use, five scaling-up productions were performed in synthetic media. This section shows the results obtained in the different fermentation processes.

Lab scale (1 L LB, in Flask)

EXPERIMENTAL CONDITIONS:

| **Stage 1:** 0.5 L Flask | |
|---|---|
| Working Volume: | 70 ml |
| Starting material: | Research Cell Bank |
| Medium: | LB broth + Ampicillin (100 $\mu$g/ml) |
| Incubation condition: | T: 37°C |
| **Stage 2:** 2 L Flask (n. 2) | |
| Working volume | 1 L (500 ml/each Flask) |
| Starting material: | Culture broth from Stage 1, start O.D. = 0.100 |
| Medium: | LB broth supplemented with Ampicillin (100 $\mu$g/ml) |
| Incubation condition: | T: 37°C |
| Induction with 1mM IPTG at: | 0.534 O.D.$_{600nm}$ (1 h 15 min) |
| Feed | Antifoam twice |

**RESULTS:**

| **Stage 1** | |
|---|---|
| final O.D.$_{600nm}$: | 6.208 after 16 h |
| **Stage 2** | |
| final O.D.$_{600nm}$: | 5.385 after 4 h |
| Proteins in the soluble bacterial extract | 126.6 mg (total amount) |
| His-UB-GAMT after the 1st Ni-affinity chromatography | 33 mg (total amount) |
| GAMT after USP2 cleavage and 2nd Ni-affinity chromatography | 20.52 mg (total amount); recovery= 62.18%* |
| Specific activity | 0.09 U/mg |
| *Dialysis performed before USP2 digestion to dilute imidazole (350 mM in the elution buffer). | |

The S.A. of the enzyme produced at lab scale level, at the end of the purification step (time 0), was lower than the previous production (0.09 vs 0.15 $\mu$mol/mg). The decrease in activity was caused by a lower degree of purity of the product than the previous one. In addition, the stability studies showed that the enzyme activity decreased of about 30% after 24 h (0.07 $\mu$mol/mg) at 37°C and then remained mostly stable until three days with values of 0.07 $\mu$mol/mg (48 h) and 0.06 $\mu$mol/mg (72 h).

**Scaling-up (5.5 L Synthetic medium, BIOREACTOR)**

Range of values obtained in 5 different scaling up processes are reported.

EXPERIMENTAL CONDITIONS:

| **Stage 1:** 1.0 L Flask | |
|---|---|
| Working Volume: | 200 ml |

(continued)

| Stage 1: 1.0 L Flask | |
|---|---|
| Starting material: | Research Cell Bank |
| Medium: | Vegetable pepton + Ampicillin (100 $\mu$g/ml) |
| Incubation condition: | T: 33°C |
| **Stage 2: BIOREACTOR** | |
| Working volume | 5.5 L |
| Starting material: | Culture broth from Stage 1, start O.D. = 0.100 |
| Medium: | Synthetic medium supplemented with Glucose-MgSO$_4$-Thiamine-Ampicillin (100 $\mu$g/ml) |
| Incubation condition: | T: 37°C |
| Induction with 1mM IPTG at: | 0.49-0.51 O.D.$_{600nm}$ |
| Feed | None |

RESULTS (see Figure 7 as a representative SDS-PAGE:

| Stage 1 | |
|---|---|
| final O.D.$_{600nm}$: | 5.9-10.0 |
| **Stage 2** | |
| final O.D.$_{600nm}$: | 0.72-1.10 |
| His-UB-GAMT after the 1st Ni-affinity chromatography | 41.86 -120 mg |
| *GAMT after USP2 cleavage and | 30.78-45 mg (total amount); recovery= 32-95.5% |
| 2nd Ni-affinity chromatography | |
| Specific activity | 0.12$\pm$0.06 U/mg |
| *Dialysis performed before USP2 digestion to dilute imidazole (350 mM in the elution buffer). | |

The scaling-up phase in the synthetic medium allowed to obtain a good yield of protein; moreover, the enzyme showed specific activity values overlapped to that obtained in LB medium at lab-scale. The aim of producing the protein in a medium suitable for the production of biological drugs was therefore achieved.

The protein storage stability at -80°C has been evaluated at different times: 0, 2, 4, 8, 12, 18, 24, 36 months. GAMT remained stable up to 4 months, then its activity decreased of 35% at 8 months, remaining at this level until to 18 months; successively, a decrease of 65% and 74% was registered after 24 and 36 months, respectively.

**Example 6 - GAA uptake by RBCs**

The ability of GAA to enter into RBCs is shown in Figure 8. A time- and concentration-dependent influx of GAA into RBCs has been observed as showed in Figure 8A: by increasing GAA concentration in the incubation medium in the range 0-100 $\mu$M, higher amounts of GAA were recovered into the cells. At GAA concentration reproducing GAA plasma content in GAMT deficient patients (approx. 50 $\mu$M as higher value), 38.42$\pm$6.4 nmol GAA/ml packed RBCs were found inside cells after 1 h of incubation at 37°C. GAA influx velocity into RBCs is perfectly linear with increasing GAA concentration, at least up to 50 $\mu$M GAA, where an influx of 0.772$\pm$0.1 nmol/min/ml RBCs at 100% Ht was observed (Figure 8 B).

**Example 7 - Evaluation of RBC SAM levels**

To carry on the study on the metabolic modeling of GAMT-loaded RBCs (see below), the cellular content of SAM has been evaluated. It was 11.7 nmol/ml of packed RBCs in agreement with Wise CK et al, 1997 (11.86 nmol/ml packed RBC). In Figure 9 a representative chromatogram of the SAM detection in RBCs is shown.

### Example 8 - RBC MAT catalytic activity

To carry on the study on the metabolic modeling of GAMT-loaded RBCs, erythrocyte MAT catalytic activity has been evaluated by measuring the incorporation of radioactivity from L-[methyl-3H] methionine into the product S-adenosyl-[methyl-3H]methionine. In the RBC lysate fraction, the MAT specific activity was 0.27 pmol/mg total proteins in agreement with Cheng H et al, 1997 (0.23 pmol/mg total protein).

### Example 9 - MAT2A cloning

Three human isoforms of MAT are present in human tissues. Of these, MAT2A is ubiquitously expressed. MAT2A forms a functional homotetramer in its active form flanked on either side by a regulatory protein, MAT2B. MAT2B regulates the activity of MAT2A by increasing the susceptibility of MAT2A to product inhibition by AdoMet but does not provide significant rate enhancement. To avoid MAT inhibition by AdoMet, we have cloned and produced only the catalytic subunits MAT2A.

Human MAT2A CDS (NCBI Reference Sequence: DQ083239.1 was successfully cloned (with Notl and Sacll restriction enzymes) into the pET45b-UB, an expression vector engineered to produce the recombinant enzyme directly fused to the C-terminus of the ubiquitin partner, cloned downstream of the vector encoded His-tag, to improve the yield and provide an easy purification of the authentic protein, without any tag (Catanzariti AM et al, 2004). The recombinant MAT precursor has been referred to as His-UB-MAT from which the NO-tag enzyme was obtained by digestion with the rHis-USP2, a deubiquitylating enzyme which removes the His-UB from the fusion product. By this strategy, MAT was expressed, purified and proved to be active. Most of the recombinant product was partitioned in the insoluble fraction (referred to as Pellet sample), however the protein was obtained starting from the soluble part since the quantity of product here present was satisfactory for our purposes and, overall, because the purification process from the soluble fraction is easier than that from inclusion bodies.

### Example 10 - Expression optimization of MAT in BL21(DE3)

Expression studies were performed at lab-scale directly in synthetic medium to optimize MAT product yield and, in the same medium, was performed the scaling-up production. This section reports the results obtained in the different fermentation processes.

Experiment N. 1: Lab-scale (2.0 L Synthetic medium, BIOREACTOR)

EXPERIMENTAL CONDITIONS:

| **Stage 1:** 0.5 L Flask | |
|---|---|
| Working Volume: | 100 ml |
| Starting material: | Research Cell Bank |
| Medium: | LB + Ampicillin (100 $\mu$g/ml) |
| Incubation condition: | T: 37°C |
| **Stage 2:** 2 L | |
| Working volume | 2 L |
| Starting material: | Culture broth from Stage 1, start O.D. = 0.1 |
| Medium: | Synthetic medium supplemented with Glucose-$MgSO_4$-Thiamine-Ampicillin |
| Incubation condition: | T: 37°C |
| Induction with 1mM IPTG at: | 0.325 O.D.$_{600nm}$ (3 h ) |
| Feed | None |

RESULTS:

| Stage 1 | |
|---|---|
| final O.D.$_{600nm}$: | 5.23 after 16 h |
| **Stage 2** | |
| final O.D.$_{600nm}$: | 0.614 after 3 h |
| Proteins in the soluble bacterial extract | 47 mg (total amount) |
| His-UB-MAT after the 1st Ni-affinity chromatography | 9 mg (total amount) |
| MAT after USP2 cleavage and 2nd Ni-affinity chromatography | 2.85 mg (total amount); recovery= 31.7% |
| Specific activity | 0.04 U/mg |

The production here obtained showed a low S.A. To optimize enzyme S.A., in the scaling-up process a DEAE chromatography was carried on instead of the Nickel IMAC.

Experiment N. 2: Scaling-up (5.5 L Synthetic medium, BIOREACTOR)

Two different scaling-up processes were performed. Values obtained are reported below.

EXPERIMENTAL CONDITIONS:

| **Stage 1:** 1.0 L Flask | |
|---|---|
| Working Volume: | 200 ml |
| Starting material: | Research Cell Bank |
| Medium: | Vegetable pepton + Ampicillin (100 $\mu$g/ml) |
| Incubation condition: | T: 33°C |
| **Stage 2:** 5 L (n. 2) | |
| Working volume | 5.5 L |
| Starting material: | Culture broth from Stage 1, start O.D. = 0.1 |
| Medium: | Synthetic medium supplemented with Glucose-MgSO$_4$-Thiamine-Ampicillin (100 $\mu$g/ml) |
| Incubation condition: | T: 37°C |
| Induction with 1mM IPTG at: | 1st process 0.49.$_{600nm}$<br>2nd process 0.51 O.D.$_{600nm}$ |
| Feed | None |

RESULTS: (see Figure 10)

| Stage 1 | |
|---|---|
| final O.D.$_{600nm}$: | 1st process 6.9; 2nd process 8.0 after 16 h |
| **Stage 2** | |
| final O.D.$_{600nm}$ after 3 h: | 1st process 1.0; 2nd process 2.0 |

(continued)

| Stage 2 | |
|---|---|
| His-UB-MAT after the 1st Ni-affinity chromatography (total amount) | 1st process 100 mg; 2nd process 200 mg |
| MAT after USP2 cleavage and 2nd DEAE chromatography (total amount) | 1st process 45 mg; recovery= 45%<br>2nd process 80 mg; recovery=40% |
| Specific activity | 1st process 0.18 U/mg; 2nd process 0.22 U/mg |

## Example 11 - Co-entrapment of GAMT and MAT into human RBCs

Human RBCs were submitted to the loading procedure both with GAMT and MAT individually and together, with defined ratios, as described in "Methods section". The amount of the entrapped proteins at the end of the procedure is shown in Table 6. As we can observe, also by starting from the same mg and units of protein (GAMT and MAT have the same specific activity), a different entrapment has been obtained, probably due to the different MW of GAMT (29 kDa) and MAT (52 kDa) and, moreover, to the fact that MAT exists as tetramer in the native form.

### Table 6: GAMT and MAT loaded into RBCs.

| | $\mu$mol/min/ml packed RBCs | |
|---|---|---|
| Sample | GAMT | MAT |
| GAMT 125 $\mu$g | 0.0128 | - |
| MAT 125 $\mu$g | - | 0.004 |
| GAMT 125 $\mu$g + MAT 125 $\mu$g | 0.0120 | 0.002 |
| GAMT 250 $\mu$g + MAT 125 $\mu$g | 0.0198 | 0.002 |
| GAMT 125 $\mu$g + MAT 250 $\mu$g | 0.0117 | 0.007 |

The ability of these engineered RBCs to metabolize L-Met and $^{13}C_2$ GAA and produce $^{13}C_2$ Cr has been evaluated by MS/MS analysis of RBC suspensions taken at different incubation times at 37°C. The results obtained show that RBCs loaded only with recombinant human GAMT or MAT were ineffective in the metabolism of extracellular GAA. Only RBCs loaded with both enzymes were able to produce $^{13}C_2$ Cr starting from GAA and Methionine and GAMT and MAT entrapped at different concentrations were capable of metabolizing $^{13}C_2$ GAA from 23 to 29% after 5h incubation at 37°C, as reported in Figure 11 (A, B and C). RBCs loaded with GAMT and MAT at the different ratios support the conclusion that a wide range of ratios could be used to metabolize GAA. From these data it could be concluded that the entire metabolic pathway from GAA to creatine is operative in RBCs loaded with recombinant human GAMT and MAT and that methionine physiologically present in human plasma can sustain the formation of creatine. The ATP needed to sustain MAT activity is provided by glucose consumption from RBC glycolysis. Again, also glucose is physiologically present in human plasma.
In conclusion, the engineered RBCs could be used in the treatment of patients with high GAA plasma concentrations, as in GAMT deficiency and/or to provide an additional biological source of creatine from said engineered RBCs. Furthermore, said RBCs could also be used in the treatment of some tumors that are sensitive to methionine blood concentrations as demonstrated by us and others (Machover D et al, 2019).

## References

Alessandri MG, Celati L, Battini R, Baldinotti F, Item C, Leuzzi V, Cioni G. HPLC assay for guanidinoacetate methyltransferase. Anal. Biochem. 2004, 331(1): 189-191.

Bailey JL. Techniques in Protein Chemistry, Elsevier Publishing Co., Amsterdam - London - New York, 1962: 340.

Bradford MM. A rapid and sensitive method for the quantitation of miCRogram quantities of protein utilizing the principle of protein-dye binding. Anal Biochem. 1976, 72: 248-54.

Catanzariti AM, Soboleva TA, Jans DA, Board PG, Baker RT. An efficient system for high-level expression and easy purification of authentic recombinant proteins. Protein Sci. 2004, 13(5):1331-9.

Carducci C, Santagata S, Leuzzi V, Carducci C, Artiola C, Giovanniello T, Battini R, Antonozzi I. Quantitative determination of guanidinoacetate and creatine in dried blood spot by flow injection analysis-electrospray tandem mass spectrometry. Clin Chim Acta. 2006, 364(1-2):180-7.

Cellarier E, Durando X, Vasson MP, Farges MC, Demiden A, Maurizis JC, Madelmont JC, Chollet P. Methionine dependency and cancer treatment. Cancer Treat Rev. 2003, 29(6):489-99.

Cheng H, Gomes-Trolin C, Aquilonius SM, Steinberg A, Löfberg C, Ekblom J, Oreland L. Levels of L-methionine S-adenosyltransferase activity in erythrocytes and concentrations of S-adenosylmethionine and S-adenosylhomocysteine in whole blood of patients with Parkinson's disease. Exp Neurol. 1997, 145(2 Pt 1): 580-585.

Clark JF & Cecil KM. Diagnostic methods and recommendations for the cerebral Creatine deficiency syndromes. Pediatr Res. 2015, 77(3): 398-405.

Dunbar M, Jaggumantri S, Sargent M, Stockler-Ipsiroglu S, van Karnebeek CD. Treatment of X-linked creatine transporter (SLC6A8) deficiency: systematic review of the literature and three new cases. Mol Genet Metab. 2014,112(4):259-74.

Iqbal F. Human guanidinoacetate n-methyl transferase (GAMT) deficiency: A treatable inborn error of metabolism. Pak J Pharm Sci. 2015, 28(6): 2207-2211.

Komoto J, Huang Y, Takata Y, Yamada T, Konishi K, Ogawa H, Gomi T, Fujioka M, Takusagawa F. Crystal structure of guanidinoacetate methyltransferase from rat liver: a model structure of protein arginine methyltransferase. J Mol Biol. 2002, 320(2): 223-35.

Leuzzi V, Rossi L, Gabucci C, Nardecchia F, Magnani M. Erythrocyte-mediated delivery of recombinant enzymes. J Inherit Metab Dis. 2016, 39(4): 519-20.

Machover D, Rossi L, Hamelin J, desterke C, Goldschmidt E, Chadefaux-Vekemans B, Bonnarme P, Briozzo P, Kopecny D, Pierigè F, Magnani M, Mollicone R, Haghghi F, Gaston-Mathè Y, Dairou J, Boucheix C, Saffroy R. Effects in cancer cells of the recombinant L-methionine Gamma-lyase from Brevibacterium aurantiacum. Encapsulation in human erythrocytes for sustained L-methionine elimination. J Pharmacol. Exp. Ther. 2019, 369: 489-502.

Magnani M, Rossi L, Bianchi M, Fornaini G, Benatti U, Guida L, Zocchi E, De Flora A. Improved metabolic properties of hexokinase-overloaded human erythrocytes. Biochim Biophys Acta. 1988, 972(1):1-8.

Mercimek-Mahmutoglu S & Salomons GS. CRatine Deficiency Syndromes. In: Adam MP, Ardinger HH, Pagon RA, Wallace SE, Bean LJH, Mefford HC, Stephens K, Amemiya A, Ledbetter N, editors. GeneReviews®. Seattle (WA): University of Washington, Seattle; 2009.

Newman EB, Budman LI, Chan EC, Greene RC, Lin RT, Woldringh CL, D'Ari R. Lack of S-adenosylmethionine results in a cell division defect in Escherichia coli. J Bacteriol. 1998, 180(14):3614-9.

Rossi L, Pierigè F, Aliano MP, Magnani M. Ongoing developments and clinical progress in drug-loaded red blood cells technologies.BioDrugs. 2020, 34: 265-272.

Shiozaki S, Shimizu C, Yamada H. Unusual Intracellular Accumulation of S-Adenosyl-L-methionine by Microorganisms. Agric. Biol. Chem. 1984; 48(9):2293-2300.

Wang W, Kramer PM, Yang S, Pereira MA, Tao L. Reversed-phase high-performance liquid chromatography procedure for the simultaneous determination of S-adenosyl-L-methionine and S-adenosyl-L-homocysteine in mouse liver and the effect of methionine on their concentrations. J. Chromatogr. B Biomed. Sci. Appl. 2001, 762(1):59-65.

Wise CK, Cooney CA, Ali SF, Poirier LA. Measuring S-adenosylmethionine in whole blood, red blood cells and cultured cells using a fast preparation method and high-performance liquid chromatography. J. Chromatogr. B Biomed. Sci. Appl. 1997, 696(1):145-152.

Sequence listing part of the description

SEQ ID NO 1 - Amino acid sequence of GAMT wild-type without the initial methionine

```
SAPSATPIFAPGENCSPAWGAAPAAYDAADTHLRILGKPVMERWETPYMHALAAAASSKG
GRVLEVGFGMAIAASKVQEAPIDEHWIIECNDGVFQRLRDWAPRQTHKVIPLKGLWEDVA
PTLPDGHFDGILYDTYPLSEETWHTHQFNFIKNHAFRLLKPGGVLTYCNLTSWGELMKSK
YSDITIMFEETQVPALLEAGFRRENIRTEVMALVPPADCRYYAFPQMITPLVTKG*
```

SEQ ID NO 2 - Amino acid sequence of GAMT CL 32 (A26N-L37M-I187Q-V215Q)

```
SAPSATPIFAPGENCSPAWGAAPANYDAADTHLRIMGKPVMERWETPYMHALAAAASSKG
GRVLEVGFGMAIAASKVQEAPIDEHWIIECNDGVFQRLRDWAPRQTHKVIPLKGLWEDVA
PTLPDGHFDGILYDTYPLSEETWHTHQFNFIKNHAFRLLKPGGVLTYCNLTSWGELMKSK
YSDITQMFEETQVPALLEAGFRRENIRTEVMALQPPADCRYYAFPQMITPLVTKG*
```

SEQ ID NO 3 - Amino acid sequence of GAMT CL 51 (L37M-I187Q-V215Q)

```
SAPSATPIFAPGENCSPAWGAAPAAYDAADTHLRIMGKPVMERWETPYMHALAAAASSKG
GRVLEVGFGMAIAASKVQEAPIDEHWIIECNDGVFQRLRDWAPRQTHKVIPLKGLWEDVA
PTLPDGHFDGILYDTYPLSEETWHTHQFNFIKNHAFRLLKPGGVLTYCNLTSWGELMKSK
YSDITQMFEETQVPALLEAGFRRENIRTEVMALQPPADCRYYAFPQMITPLVTKG*
```

SEQ ID NO 4 - Amino acid sequence of GAMT CL 76 (A26N-L371-I187Q-V215Q)

```
SAPSATPIFAPGENCSPAWGAAPANYDAADTHLRIIGKPVMERWETPYMHALAAAASSKG
GRVLEVGFGMAIAASKVQEAPIDEHWIIECNDGVFQRLRDWAPRQTHKVIPLKGLWEDVA
PTLPDGHFDGILYDTYPLSEETWHTHQFNFIKNHAFRLLKPGGVLTYCNLTSWGELMKSK
YSDITQMFEETQVPALLEAGFRRENIRTEVMALQPPADCRYYAFPQMITPLVTKG*
```

Important Note: the amino acid numbering refers to the full-length GAMT, including the starting Methionine (M1), which is not expressed by these constructs (because it is no longer present in the mature form).

SEQ ID NO 5 - Amino acid sequence of MAT2A

```
MNGQLNGFHEAFIEEGTFLFTSESVGEGHPDKICDQISDAVLDAHLQQDPDAKVACETVAKTGMILLAGE
ITSRAAVDYQKVVREAVKHIGYDDSSKGFDYKTCNVLVALEQQSPDIAQGVHLDRNEEDIGAGDQGLMFG
YATDETEECMPLTIVLAHKLNAKLAELRRNGTLPWLRPDSKTQVTVQYMQDRGAVLPIRVHTIVISVQHD
EEVCLDEMRDALKEKVIKAVVPAKYLDEDTIYHLQPSGRFVIGGPQGDAGLTGRKIIVDTYGGWGAHGGG

AFSGKDYTKVDRSAAYAARWVAKSLVKGGLCRRVLVQVSYAIGVSHPLSISIFHYGTSQKSERELLEIVK
KNFDLRPGVIVRDLDLKKPIYQRTAAYGHFGRDSFPWEVPKKLKY
```

SEQ ID NO 6 - Nucleotide sequence of His-UB-GAMT wild-type

ATGGCACATCACCACCACCATCACGTGATGCAGATCTTCGTGAAGACCCTGACTGGTAAGACCATCACTC
TCGAGGTGGAGCCGAGTGACACCATTGAGAATGTCAAGGCAAAGATCCAAGACAAGGAAGGCATCCCTCC
TGACCAGCAGAGGTTGATCTTTGCTGGGAAACAGCTGGAAGATGGACGCACCCTGTCTGACTACAACATC
CAGAAAGAGTCCACCCTGCACCTGGTGCTCCGTCTCCGCGGTGGATCGGCCCCCAGCGCGACCCCCATCT
TCGCGCCCGGCGAGAACTGCAGCCCCGCGTGGGGGGCGGCGCCCGCG**GCC**TACGACGCAGCGGACACGCA
CCTGCGCATC**CTG**GGCAAGCCGGTGATGGAGCGCTGGGAGACCCCCTATATGCACGCGCTGGCCGCCGCC
GCCTCCTCCAAAGGGGGCCGGGTCCTGGAGGTGGGCTTTGGCATGGCCATCGCAGCGTCAAAGGTGCAGG
AGGCGCCCATTGATGAGCATTGGATCATCGAGTGCAATGACGGCGTCTTCCAGCGGCTCCGGGACTGGGC
CCCACGGCAGACACACAAGGTCATCCCCTTGAAAGGCCTGTGGGAGGATGTGGCACCCACCCTGCCTGAC
GGTCACTTTGATGGGATCCTGTACGACACGTACCCACTCTCGGAGGAGACCTGGCACACACACCAGTTCA
ACTTCATCAAGAACCACGCCTTTCGCCTGCTGAAGCCGGGGGGCGTCCTCACCTACTGCAACCTCACCTC
CTGGGGGGAGCTGATGAAGTCCAAGTACTCAGACATCACC**ATC**ATGTTTGAGGAGACGCAGGTGCCCGCG
CTGCTGGAGGCCGGCTTCCGGAGGGAGAACATCCGTACGGAGGTGATGGCGCTG**GTC**CCACCGGCCGACT
GCCGCTACTACGCCTTCCCACAGATGATCACGCCCCTGGTGACCAAAGGCTGA

SEQ ID NO 7 - Nucleotide sequence of His-UB-GAMT mutant CLONE 32 (A26N-L37M-I187Q-V215Q)

ATGGCACATCACCACCACCATCACGTGATGCAGATCTTCGTGAAGACCCTGACTGGTAAGACCATCACTC
TCGAGGTGGAGCCGAGTGACACCATTGAGAATGTCAAGGCAAAGATCCAAGACAAGGAAGGCATCCCTCC
TGACCAGCAGAGGTTGATCTTTGCTGGGAAACAGCTGGAAGATGGACGCACCCTGTCTGACTACAACATC
CAGAAAGAGTCCACCCTGCACCTGGTGCTCCGTCTCCGCGGTGGATCGGCCCCCAGCGCGACCCCCATCT
TCGCGCCCGGCGAGAACTGCAGCCCCGCGTGGGGGGCGGCGCCCGCG**AAC**TACGACGCAGCGGACACGCA
CCTGCGCATC**ATG**GGCAAGCCGGTGATGGAGCGCTGGGAGACCCCCTATATGCACGCGCTGGCCGCCGCC
GCCTCCTCCAAAGGGGGCCGGGTCCTGGAGGTGGGCTTTGGCATGGCCATCGCAGCGTCAAAGGTGCAGG
AGGCGCCCATTGATGAGCATTGGATCATCGAGTGCAATGACGGCGTCTTCCAGCGGCTCCGGGACTGGGC
CCCACGGCAGACACACAAGGTCATCCCCTTGAAAGGCCTGTGGGAGGATGTGGCACCCACCCTGCCTGAC
GGTCACTTTGATGGGATCCTGTACGACACGTACCCACTCTCGGAGGAGACCTGGCACACACACCAGTTCA
ACTTCATCAAGAACCACGCCTTTCGCCTGCTGAAGCCGGGGGGCGTCCTCACCTACTGCAACCTCACCTC
CTGGGGGGAGCTGATGAAGTCCAAGTACTCAGACATCACC**CAG**ATGTTTGAGGAGACGCAGGTGCCCGCG
CTGCTGGAGGCCGGCTTCCGGAGGGAGAACATCCGTACGGAGGTGATGGCGCTG**CAG**CCACCGGCCGACT
GCCGCTACTACGCCTTCCCACAGATGATCACGCCCCTGGTGACCAAAGGCTGA

SEQ ID NO 8 - Nucleotide sequence of His-UB-GAMT mutant CLONE 51 (L37M-I187Q-V215Q)

ATGGCACATCACCACCACCATCACGTGATGCAGATCTTCGTGAAGACCCTGACTGGTAAGACCATCACTC
TCGAGGTGGAGCCGAGTGACACCATTGAGAATGTCAAGGCAAAGATCCAAGACAAGGAAGGCATCCCTCC
TGACCAGCAGAGGTTGATCTTTGCTGGGAAACAGCTGGAAGATGGACGCACCCTGTCTGACTACAACATC
CAGAAAGAGTCCACCCTGCACCTGGTGCTCCGTCTCCGCGGTGGATCGGCCCCCAGCGCGACCCCCATCT
TCGCGCCCGGCGAGAACTGCAGCCCCGCGTGGGGGGCGGCGCCCGCGGCCTACGACGCAGCGGACACGCA
CCTGCGCATC**ATG**GGCAAGCCGGTGATGGAGCGCTGGGAGACCCCCTATATGCACGCGCTGGCCGCCGCC
GCCTCCTCCAAAGGGGGCCGGGTCCTGGAGGTGGGCTTTGGCATGGCCATCGCAGCGTCAAAGGTGCAGG
AGGCGCCCATTGATGAGCATTGGATCATCGAGTGCAATGACGGCGTCTTCCAGCGGCTCCGGGACTGGGC
CCCACGGCAGACACACAAGGTCATCCCCTTGAAAGGCCTGTGGGAGGATGTGGCACCCACCCTGCCTGAC
GGTCACTTTGATGGGATCCTGTACGACACGTACCCACTCTCGGAGGAGACCTGGCACACACACCAGTTCA
ACTTCATCAAGAACCACGCCTTTCGCCTGCTGAAGCCGGGGGGCGTCCTCACCTACTGCAACCTCACCTC
CTGGGGGGAGCTGATGAAGTCCAAGTACTCAGACATCACC**CAG**ATGTTTGAGGAGACGCAGGTGCCCGCG
CTGCTGGAGGCCGGCTTCCGGAGGGAGAACATCCGTACGGAGGTGATGGCGCTG**CAG**CCACCGGCCGACT
GCCGCTACTACGCCTTCCCACAGATGATCACGCCCCTGGTGACCAAAGGCTGA

SEQ ID NO 9 - Nucleotide sequence of His-UB-GAMT mutant CLONE 76 (A26N-L37I-I187Q-V215Q)

ATGGCACATCACCACCACCATCACGTGATGCAGATCTTCGTGAAGACCCTGACTGGTAAGACCATCACTC
TCGAGGTGGAGCCGAGTGACACCATTGAGAATGTCAAGGCAAAGATCCAAGACAAGGAAGGCATCCCTCC
TGACCAGCAGAGGTTGATCTTTGCTGGGAAACAGCTGGAAGATGGACGCACCCTGTCTGACTACAACATC
CAGAAAGAGTCCACCCTGCACCTGGTGCTCCGTCTCCGCGGTGGATCGGCCCCCAGCGCGACCCCCATCT
TCGCGCCCGGCGAGAACTGCAGCCCCGCGTGGGGGGCGGCGCCCGCG**AAC**TACGACGCAGCGGACACGCA
CCTGCGCATC**ATT**GGCAAGCCGGTGATGGAGCGCTGGGAGACCCCCTATATGCACGCGCTGGCCGCCGCC
GCCTCCTCCAAAGGGGGCCGGGTCCTGGAGGTGGGCTTTGGCATGGCCATCGCAGCGTCAAAGGTGCAGG
AGGCGCCCATTGATGAGCATTGGATCATCGAGTGCAATGACGGCGTCTTCCAGCGGCTCCGGGACTGGGC
CCCACGGCAGACACACAAGGTCATCCCCTTGAAAGGCCTGTGGGAGGATGTGGCACCCACCCTGCCTGAC
GGTCACTTTGATGGGATCCTGTACGACACGTACCCACTCTCGGAGGAGACCTGGCACACACACCAGTTCA
ACTTCATCAAGAACCACGCCTTTCGCCTGCTGAAGCCGGGGGGCGTCCTCACCTACTGCAACCTCACCTC
CTGGGGGGAGCTGATGAAGTCCAAGTACTCAGACATCACC**CAG**ATGTTTGAGGAGACGCAGGTGCCCGCG
CTGCTGGAGGCCGGCTTCCGGAGGGAGAACATCCGTACGGAGGTGATGGCGCTG**CAG**CCACCGGCCGACT
GCCGCTACTACGCCTTCCCACAGATGATCACGCCCCTGGTGACCAAAGGCTGA

SEQ ID NO 10 - Nucleotide sequence of MAT2A wild-type

ATGAACGGACAGCTCAACGGCTTCCACGAGGCGTTCATCGAGGAGGGCACATTCCTTTTCACCTCAGAGT
CGGTCGGGGAAGGCCACCCAGATAAGATTTGTGACCAAATCAGTGATGCTGTCCTTGATGCCCACCTTCA
GCAGGATCCTGATGCCAAAGTAGCTTGTGAAACTGTTGCTAAAACTGGAATGATCCTTCTTGCTGGGGAA
ATTACATCCAGAGCTGCTGTTGACTACCAGAAAGTGGTTCGTGAAGCTGTTAAACACATTGGATATGATG
ATTCTTCCAAAGGTTTTGACTACAAGACTTGTAACGTGCTGGTAGCCTTGGAGCAACAGTCACCAGATAT
TGCTCAAGGTGTTCATCTTGACAGAAATGAAGAAGACATTGGTGCTGGAGACCAGGGCTTAATGTTTGGC
TATGCCACTGATGAAACTGAGGAGTGTATGCCTTTAACCATTGTCTTGGCACACAAGCTAAATGCCAAAC
TGGCAGAACTACGCCGTAATGGCACTTTGCCTTGGTTACGCCCTGATTCTAAAACTCAAGTTACTGTGCA
GTATATGCAGGATCGAGGTGCTGTGCTTCCCATCAGAGTCCACACAATTGTTATATCTGTTCAGCATGAT
GAAGAGGTTTGTCTTGATGAAATGAGGGATGCCCTAAAGGAGAAAGTCATCAAAGCAGTTGTGCCTGCGA
AATACCTTGATGAGGATACAATCTACCACCTACAGCCAAGTGGCAGATTTGTTATTGGTGGGCCTCAGGG
TGATGCTGGTTTGACTGGACGGAAAATCATTGTGGACACTTATGGCGGTTGGGGTGCTCATGGAGGAGGT
GCCTTTTCAGGAAAGGATTATACCAAGGTCGACCGTTCAGCTGCTTATGCTGCTCGTTGGGTGGCAAAAT
CCCTTGTTAAAGGAGGTCTGTGCCGGAGGGTTCTTGTTCAGGTCTCTTATGCTATTGGAGTTTCTCATCC
ATTATCTATCTCCATTTTCCATTATGGTACCTCTCAGAAGAGTGAGAGAGAGCTATTAGAGATTGTGAAG
AAGAATTTCGATCTCCGCCCTGGGGTCATTGTCAGGGATCTGGATCTGAAGAAGCCAATTTATCAGAGGA
CTGCAGCCTATGGCCACTTTGGTAGGGACAGCTTCCCATGGGAAGTGCCCAAAAAGCTTAAATATTGA

SEQ ID NO 11 - Nucleotide sequence of His-UB-MAT2A

ATGGCACATCACCACCACCATCACGTGATGCAGATCTTCGTGAAGACCCTGACTGGTAAGACCATCACTC
TCGAGGTGGAGCCGAGTGACACCATTGAGAATGTCAAGGCAAAGATCCAAGACAAGGAAGGCATCCCTCC
TGACCAGCAGAGGTTGATCTTTGCTGGGAAACAGCTGGAAGATGGACGCACCCTGTCTGACTACAACATC
CAGAAAGAGTCCACCCTGCACCTGGTGCTCCGTCTCCGCGGTGGAATGAACGGACAGCTCAACGGCTTCC
ACGAGGCGTTCATCGAGGAGGGCACATTCCTTTTCACCTCAGAGTCGGTCGGGGAAGGCCACCCAGATAA
GATTTGTGACCAAATCAGTGATGCTGTCCTTGATGCCCACCTTCAGCAGGATCCTGATGCCAAAGTAGCT
TGTGAAACTGTTGCTAAAACTGGAATGATCCTTCTTGCTGGGGAAATTACATCCAGAGCTGCTGTTGACT
ACCAGAAAGTGGTTCGTGAAGCTGTTAAACACATTGGATATGATGATTCTTCCAAAGGTTTTGACTACAA
GACTTGTAACGTGCTGGTAGCCTTGGAGCAACAGTCACCAGATATTGCTCAAGGTGTTCATCTTGACAGA
AATGAAGAAGACATTGGTGCTGGAGACCAGGGCTTAATGTTTGGCTATGCCACTGATGAAACTGAGGAGT
GTATGCCTTTAACCATTGTCTTGGCACACAAGCTAAATGCCAAACTGGCAGAACTACGCCGTAATGGCAC
TTTGCCTTGGTTACGCCCTGATTCTAAAACTCAAGTTACTGTGCAGTATATGCAGGATCGAGGTGCTGTG
CTTCCCATCAGAGTCCACACAATTGTTATATCTGTTCAGCATGATGAAGAGGTTTGTCTTGATGAAATGA

GGGATGCCCTAAAGGAGAAAGTCATCAAAGCAGTTGTGCCTGCGAAATACCTTGATGAGGATACAATCTA
CCACCTACAGCCAAGTGGCAGATTTGTTATTGGTGGGCCTCAGGGTGATGCTGGTTTGACTGGACGGAAA
ATCATTGTGGACACTTATGGCGGTTGGGGTGCTCATGGAGGAGGTGCCTTTTCAGGAAAGGATTATACCA
AGGTCGACCGTTCAGCTGCTTATGCTGCTCGTTGGGTGGCAAAATCCCTTGTTAAAGGAGGTCTGTGCCG
GAGGGTTCTTGTTCAGGTCTCTTATGCTATTGGAGTTTCTCATCCATTATCTATCTCCATTTTCCATTAT
GGTACCTCTCAGAAGAGTGAGAGAGAGCTATTAGAGATTGTGAAGAAGAATTTCGATCTCCGCCCTGGGG
TCATTGTCAGGGATCTGGATCTGAAGAAGCCAATTTATCAGAGGACTGCAGCCTATGGCCACTTTGGTAG
GGACAGCTTCCCATGGGAAGTGCCCAAAAAGCTTAAATATTGA

SEQ ID NO 12 - GAMT PmlI_Fwd cloning primer
ACGGTACTTCACGTGATGAGCGCCCCCAGCGCGA

SEQ ID NO 13 - UB-GAMT NcoI_Fwd cloning primer
ACGGTACTTCCATGGGCCCCCAGCGCGACCCCC

SEQ ID NO 14 - GAMT HindIII_Rev cloning primer
GGATACCTAAAGCTTCAGCCTTTGGTCACCAGGGGCG

SEQ ID NO 15 - UB-GAMT (L37I) mutagenesis primer
CACGCACCTGCGCATC**ATT**GGCAAGCCGGTGATGG

SEQ ID NO 16 - UB-GAMT (G38A) mutagenesis primer
ACCTGCGCATCCTGG**C**CAAGCCGGTGATGGAGC

SEQ ID NO 17 - UB-GAMT (L37I - G38A) mutagenesis primer
CACGCACCTGCGCATC**ATTGC**CAAGCCGGTGATGGAGC

SEQ ID NO 18 - UB-GAMT (L37M) mutagenesis primer
ACGCACCTGCGCATC**A**TGGGCAAGCCGGTGATGG

SEQ ID NO 19 - UB-GAMT (I187Q) mutagenesis primer
GTACTCAGACATCACC**CAG**ATGTTTGAGGAGACGCAGGTG

SEQ ID NO 20 - UB-GAMT (A26N) mutagenesis primer
GGGCGGCGCCCGCG**AA**CTACGACGCAGCGGAC

SEQ ID NO 21 - UB-GAMT (V215Q) mutagenesis primer
GGAGGTGATGGCGCTG**CAG**CCACCGGCCGACTGCC

SEQ ID NO 22 - GAMT 7*_Fwd sequencing primer
GCCCCCAGCGCGACCCCCATCT

SEQ ID NO 23 - GAMT 312*_Fwd sequencing primer
ACGGCAGACACACAAGGTCATCC

SEQ ID NO 24 - GAMT 711*_Rev sequencing primer
TCAGCCTTTGGTCACCAGGGGC

SEQ ID NO 25 - T7 promoter sequencing primer
TAATACGACTCACTATAGGG

SEQ ID NO 26 - T7 terminator sequencing primer
GCTAGTTATTGCTCAGCGG

SEQ ID NO 27 - MAT2A Sacll_Fwd cloning primer
CGTCTCCGCGGTGGAATGAACGGACAGCTCAACGGC

SEQ ID NO 28 - MAT2A Notl_Rev cloning primer
CGTCTGCGGCCGCTCAATATTTAAGCTTTTTGGGCACTTCCC

SEQ ID NO 29 - MAT2A 100*_Fwd sequencing primer
GTGACCAAATCAGTGATGCTGTCC

SEQ ID NO 30 - MAT2A 398*_Fwd sequencing primer
AGACCAGGGCTTAATGTTTGGC

SEQ ID NO 31 - MAT2A 799*_Fwd sequencing primer
TTGTGGACACTTATGGCGGTTG

SEQ ID NO 32 - Amino acid sequence of GAMT wild-type containing the starting methionine

MSAPSATPIFAPGENCSPAWGAAPAAYDAADTHLRILGKPVMERWETPYMHA
LAAAASSKGGRVLEVGFGMAIAASKVQEAPIDEHWIIECNDGVFQRLRDWAPR
QTHKVIPLKGLWEDVAPTLPDGHFDGILYDTYPLSEETWHTHQFNFIKNHAFRL
LKPGGVLTYCNLTSWGELMKSKYSDITIMFEETQVPALLEAGFRRENIRTEVMA
LVPPADCRYYAFPQMITPLVTKG*

## Claims

1. Isolated cell modified for catalyzing the conversion of guanidinoacetate acid (GAA) into creatine in the presence of glucose and methionine comprising an exogenous guanidinoacetate methyltransferase (GAMT) and methionine adenosyltransferase (MAT) protein.

2. Isolated cell according to claim 1, wherein the cell is a human cell.

3. Isolated cell according to claim 1 or 2, wherein the cell is selected from erythrocyte, hepatocyte, pancreas cell.

4. Isolated cell according to any of the preceding claims, wherein said cell lacks or exhibits a reduced endogenous GAMT and/or MAT enzymatic activity.

5. Isolated cell according to any of the preceding claims, wherein said cell is isolated from a subject suffering of Guanidinoacetate methyltransferase (GAMT) deficiency.

6. Isolated cell according to any of the preceding claims, wherein said cell is loaded with said GAMT protein, a fragment or a variant thereof, and with said MAT protein, a fragment or a variant thereof, in particular wherein said MAT is the catalytic subunit MAT2A, wherein said a fragment or a variant thereof having the same or improved enzymatic activity of the wild-type protein.

7. Isolated cell according to any of preceding claims, wherein said cell is transformed or transfected with exogenous nucleic acids comprising at least a nucleic acid sequence encoding said GAMT protein, a fragment or a variant thereof, and a nucleic acid sequence encoding at least said MAT protein, a fragment or a variant thereof, wherein said a fragment or a variant thereof having the same or improved enzymatic activity of the wild-type protein.

8. Isolated cell according to any of the preceding claims, wherein said GAMT and MAT are the naturally or recombinant human GAMT and MAT, in particular wherein said MAT is the catalytic subunit MAT2A.

9. Isolated cell according to any of the preceding claims, wherein said GAMT is the wild-type GAMT having the sequence SEQ ID NO 1 wherein one or more of the following mutations are inserted in the sequence of said protein: A26N, L37M, I187Q and V215Q, preferably wherein all of said mutations are inserted.

10. Isolated cell according to any of preceding claims, wherein said GAMT is the GAMT having the SEQ ID NO 1, SEQ ID NO 2, SEQ ID NO 3, SEQ ID NO 4 or SEQ ID NO 32 and said MAT is the MAT2A having the SEQ ID NO 5, or their variants with at least 98%, preferably 99% of identities.

11. Isolated cell according to any of the preceding claims, wherein said GAMT protein, a fragment or variant thereof, and said MAT protein, a fragment or variant thereof, are present in said cell in a ratio ranging from 10:1 to 1:10.

12. Isolated cell according to any of preceding claims for use as a medicament.

13. Isolated cell according to any of preceding claims, for use in the treatment of a subject suffering from a disease or condition caused by and/or **characterized by** a deficit of creatine or in the treatment of GAMT deficiency, creatine transporter deficiency, methionine-dependent tumors.

14. A pharmaceutical composition comprising a plurality of modified cells according to any one of claims 1 to 13 and one or more carriers and/or diluent, in particular for use in the treatment of GAMT deficiency, creatine transporter deficiency, methionine-dependent tumors.

15. An *in vitro* method for the preparation of a modified cell, whereby said cell is capable of catalyzing the conversion of guanidinoacetate acid (GAA) into creatine in the presence of glucose and methionine, comprising a step of modifying a cell using an exogenous guanidinoacetate methyltransferase (GAMT) and methionine adenosyltransferase (MAT) protein, wherein the exogenous guanidinoacetate methyltransferase (GAMT) and methionine adenosyltransferase (MAT) protein are inserted or loaded or transfected into the cell.


**Patentansprüche**

1. Isolierte Zelle, die für die Katalyse der Umwandlung von Guanidinoacetatsäure (GAA) in Kreatin in Gegenwart von Glucose und Methionin modifiziert wurde und ein exogenes Guanidinoacetat-Methyltransferase- (GAMT-) und Methionin-Adenosyltransferase- (MAT-) Protein umfasst.

2. Isolierte Zelle nach Anspruch 1, wobei die Zelle eine menschliche Zelle ist.

3. Isolierte Zelle nach Anspruch 1 oder 2, wobei die Zelle aus Erythrozyt, Hepatozyt, Pankreaszelle ausgewählt ist.

4. Isolierte Zelle nach einem der vorstehenden Ansprüche, wobei die Zelle keine oder eine verringerte endogene GAMT- und/oder MAT-Enzymaktivität aufweist.

5. Isolierte Zelle nach einem der vorstehenden Ansprüche, wobei die Zelle aus einem Subjekt isoliert wurde, das an einem Mangel an Guanidinoacetat-Methyltransferase (GAMT) leidet.

6. Isolierte Zelle nach einem der vorstehenden Ansprüche, wobei die Zelle mit dem GAMT-Protein, einem Fragment oder einer Variante davon und mit dem MAT-Protein, einem Fragment oder einer Variante davon beladen ist, wobei es sich bei dem MAT insbesondere um die katalytische Untereinheit MAT2A handelt, wobei das Fragment oder die Variante davon die gleiche oder eine verbesserte Enzymaktivität des Wildtyp-Proteins aufweist.

7. Isolierte Zelle nach einem der vorstehenden Ansprüche, wobei die Zelle mit exogenen Nukleinsäuren transformiert oder transfiziert ist, umfassend mindestens eine Nukleinsäuresequenz, die das GAMT-Protein, ein Fragment oder eine Variante davon kodiert, und eine Nukleinsäuresequenz, die mindestens das MAT-Protein, ein Fragment oder eine Variante davon kodiert, wobei das Fragment oder die Variante davon die gleiche oder eine verbesserte Enzymaktivität des Wildtyp-Proteins aufweist.

8. Isolierte Zelle nach einem der vorstehenden Ansprüche, wobei die GAMT und MAT natürliche oder rekombinante humane GAMT und MAT sind, wobei MAT insbesondere die katalytische Untereinheit MAT2A ist.

9. Isolierte Zelle nach einem der vorstehenden Ansprüche, wobei die GAMT die Wildtyp-GAMT mit der Sequenz SEQ ID NO 1 ist, wobei eine oder mehrere der folgenden Mutationen in die Sequenz des Proteins eingefügt sind: A26N, L37M, I187Q und V215Q, wobei vorzugsweise alle genannten Mutationen eingefügt sind.

10. Isolierte Zelle nach einem der vorstehenden Ansprüche, wobei die GAMT die GAMT mit der SEQ ID NO 1, SEQ ID NO 2, SEQ ID NO 3, SEQ ID NO 4 oder SEQ ID NO 32 ist und die MAT die MAT2A mit der SEQ ID NO 5 ist, oder deren Varianten mit mindestens 98 %, vorzugsweise 99 %, Identität.

11. Isolierte Zelle nach einem der vorstehenden Ansprüche, wobei das GAMT-Protein, ein Fragment oder eine Variante davon, und das MAT-Protein, ein Fragment oder eine Variante davon in der Zelle in einem Verhältnis im Bereich von 10:1 bis 1:10 vorhanden sind.

12. Isolierte Zelle nach einem der vorstehenden Ansprüche zur Verwendung als Medikament.

13. Isolierte Zelle nach einem der vorstehenden Ansprüche zur Verwendung bei der Behandlung eines Subjekts, das an einer Krankheit oder einem Zustand leidet, verursacht durch und/oder **gekennzeichnet durch** einen Kreatinmangel, oder bei der Behandlung von GAMT-Mangel, Kreatintransportermangel, methioninabhängigen Tumoren.

14. Pharmazeutische Zusammensetzung, umfassend eine Vielzahl modifizierter Zellen nach einem der Ansprüche 1 bis 13 und einen oder mehrere Träger und/oder Verdünnungsmittel, insbesondere zur Verwendung bei der Behandlung von GAMT-Mangel, Kreatintransportermangel und methioninabhängigen Tumoren.

15. *In-vitro-Verfahren* zur Herstellung einer modifizierten Zelle, wobei die Zelle in der Lage ist, die Umwandlung von Guanidinoacetatsäure (GAA) in Kreatin in Gegenwart von Glucose und Methionin zu katalysieren, umfassend einen Schritt der Modifizierung einer Zelle unter Verwendung eines exogenen Guanidinoacetat-Methyltransferase- (GAMT-) und Methionin-Adenosyltransferase- (MAT-) Proteins, wobei das exogene Guanidinoacetat-Methyltransferase- (GAMT-) und Methionin-Adenosyltransferase- (MAT-) Protein in die Zelle eingeführt, geladen oder transfiziert wird.

## Revendications

1. Cellule isolée modifiée pour la catalyse de la conversion de l'acide guanidinoacétique (GAA) en créatine en présence de glucose et de méthionine, comprenant une protéine exogène de type guanidinoacétate méthyltransférase (GAMT) et une protéine exogène de type méthionine adénosyltransférase (MAT).

2. Cellule isolée selon la revendication 1, dans laquelle la cellule est une cellule humaine.

3. Cellule isolée selon la revendication 1 ou 2, dans laquelle la cellule est choisie parmi érythrocyte, hépatocyte ou cellule du pancréas.

4. Cellule isolée selon l'une quelconque des revendications précédentes, dans laquelle ladite cellule est dépourvue d'activité enzymatique endogène GAMT et/ou MAT ou présente une activité enzymatique endogène GAMT et/ou MAT réduite.

5. Cellule isolée selon l'une quelconque des revendications précédentes, dans laquelle ladite cellule est isolée à partir d'un sujet souffrant d'un déficit en guanidinoacetate méthyltransférase (GAMT).

6. Cellule isolée selon l'une quelconque des revendications précédentes, dans laquelle ladite cellule est chargée de ladite protéine GAMT, d'un fragment ou d'une variante de celle-ci, et de ladite protéine MAT, d'un fragment ou d'une variante de celle-ci, en particulier dans laquelle ladite MAT est la sous-unité catalytique MAT2A, dans laquelle ledit fragment ou ladite variante de celle-ci a une activité enzymatique identique ou améliorée par rapport à celle de la protéine de type sauvage.

7. Cellule isolée selon l'une quelconque des revendications précédentes, dans laquelle ladite cellule est transformée ou transfectée avec des acides nucléiques exogènes comprenant au moins une séquence d'acides nucléiques codant pour ladite protéine GAMT, un fragment ou une variante de celle-ci, et une séquence d'acides nucléiques codant au moins pour ladite protéine MAT, un fragment ou une variante de celle-ci, dans laquelle ledit fragment ou ladite variante de celle-ci a une activité enzymatique identique ou améliorée par rapport à celle de la protéine de type sauvage.

8. Cellule isolée selon l'une quelconque des revendications précédentes, dans laquelle ladite GAMT et ladite MAT sont la GAMT et la MAT humaines naturelles ou recombinantes, en particulier dans laquelle ladite MAT est la sous-unité catalytique MAT2A.

9. Cellule isolée selon l'une quelconque des revendications précédentes, dans laquelle ladite GAMT est la GAMT de type sauvage ayant la séquence SEQ ID NO 1 dans laquelle une ou plusieurs des mutations suivantes sont insérées dans la séquence de ladite protéine : A26N, L37M, 1187Q et V215Q, de préférence dans laquelle toutes lesdites mutations sont insérées.

10. Cellule isolée selon l'une quelconque des revendications précédentes, dans laquelle ladite GAMT est la GAMT ayant le SEQ ID NO 1, SEQ ID NO 2, SEQ ID NO 3, SEQ ID NO 4 ou SEQ ID NO 32 et ladite MAT est la MAT2A ayant le SEQ ID NO 5, ou leurs variantes avec au moins 98 %, de préférence 99 % d'identités.

11. Cellule isolée selon l'une quelconque des revendications précédentes, dans laquelle ladite protéine GAMT, un fragment ou une variante de celle-ci, et ladite protéine MAT, un fragment ou une variante de celle-ci, sont présents dans ladite cellule dans un rapport allant de 10:1 à 1:10.

12. Cellule isolée selon l'une quelconque des revendications précédentes destinée à être utilisée en tant que médicament.

13. Cellule isolée selon l'une quelconque des revendications précédentes, destinée à être utilisée dans le traitement d'un sujet souffrant d'une maladie ou d'un problème de santé causé et/ou **caractérisé par** un déficit en créatine, ou dans le traitement d'un déficit en GAMT, d'un déficit en transporteur de la créatine ou de tumeurs dépendantes de la méthionine.

14. Composition pharmaceutique comprenant une pluralité de cellules modifiées selon l'une quelconque des revendications 1 à 13 et un ou plusieurs supports et/ou diluants, en particulier destinée à être utilisée dans le traitement d'un déficit en GAMT, d'un déficit en transporteur de la créatine ou de tumeurs dépendantes de la méthionine.

15. Procédé *in vitro* pour la préparation d'une cellule modifiée, dans lequel ladite cellule est capable de catalyser la conversion de l'acide guanidinoacétique (GAA) en créatine en présence de glucose et de méthionine, comprenant une étape de modification d'une cellule à l'aide d'une protéine exogène de type guanidinoacétate méthyltransférase (GAMT) et d'une protéine exogène de type méthionine adénosyltransférase (MAT), dans lequel la protéine exogène de type guanidinoacétate méthyltransférase (GAMT) et la protéine exogène de type méthionine adénosyltransférase (MAT) sont insérées ou chargées ou transfectées dans la cellule.

FIGURE 1.

FIGURE 2.

FIGURE 3.

FIGURE 4.

FIGURE 5.

FIGURE 6.

FIGURE 7.

FIGURE 8.

FIGURE 9.

[A]

[B]

1. Loaded
2. Unbound
3. Wash ( 10 mM imidazole)
4. Elution (200 mM imidazole)
5. Elution (200 mM imidazole)
6. **MAT**

FIGURE 10.

FIGURE 11.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

• US 10849858 B

### Non-patent literature cited in the description

• **ALESSANDRI MG ; CELATI L ; BATTINI R ; BALDINOTTI F ; ITEM C ; LEUZZI V ; CIONI G**. HPLC assay for guanidinoacetate methyltransferase.. *Anal. Biochem.*, 2004, vol. 331 (1), 189-191

• **BAILEY JL**. Techniques in Protein Chemistry. Elsevier Publishing Co, 1962, 340

• **BRADFORD MM.** A rapid and sensitive method for the quantitation of miCRogram quantities of protein utilizing the principle of protein-dye binding.. *Anal Biochem.*, 1976, vol. 72, 248-54

• **CATANZARITI AM ; SOBOLEVA TA ; JANS DA ; BOARD PG ; BAKER RT**. An efficient system for high-level expression and easy purification of authentic recombinant proteins. *Protein Sci.*, 2004, vol. 13 (5), 1331-9

• **CARDUCCI C ; SANTAGATA S ; LEUZZI V ; CARDUCCI C ; ARTIOLA C ; GIOVANNIELLO T ; BATTINI R ; ANTONOZZI I**. Quantitative determination of guanidinoacetate and creatine in dried blood spot by flow injection analysis-electrospray tandem mass spectrometry. *Clin Chim Acta.*, 2006, vol. 364 (1-2), 180-7

• **CELLARIER E ; DURANDO X ; VASSON MP ; FARGES MC ; DEMIDEN A ; MAURIZIS JC ; MADELMONT JC ; CHOLLET P**. Methionine dependency and cancer treatment. *Cancer Treat Rev.*, 2003, vol. 29 (6), 489-99

• **CHENG H ; GOMES-TROLIN C ; AQUILONIUS SM ; STEINBERG A ; LÖFBERG C ; EKBLOM J ; ORELAND L**. Levels of L-methionine S-adenosyltransferase activity in erythrocytes and concentrations of S-adenosylmethionine and S-adenosylhomocysteine in whole blood of patients with Parkinson's disease. *Exp Neurol.*, 1997, vol. 145, 580-585

• **CLARK JF ; CECIL KM.** Diagnostic methods and recommendations for the cerebral Creatine deficiency syndromes.. *Pediatr Res.*, 2015, vol. 77 (3), 398-405

• **DUNBAR M ; JAGGUMANTRI S ; SARGENT M ; STOCKLER-IPSIROGLU S ; VAN KARNEBEEK CD**. Treatment of X-linked creatine transporter (SLC6A8) deficiency: systematic review of the literature and three new cases.. *Mol Genet Metab.*, 2014, vol. 112 (4), 259-74

• **IQBAL F**. Human guanidinoacetate n-methyl transferase (GAMT) deficiency: A treatable inborn error of metabolism.. *Pak J Pharm Sci.*, 2015, vol. 28 (6), 2207-2211

• **KOMOTO J ; HUANG Y ; TAKATA Y ; YAMADA T ; KONISHI K ; OGAWA H ; GOMI T ; FUJIOKA M ; TAKUSAGAWA F**. Crystal structure of guanidinoacetate methyltransferase from rat liver: a model structure of protein arginine methyltransferase.. *J Mol Biol.*, 2002, vol. 320 (2), 223-35

• **LEUZZI V ; ROSSI L ; GABUCCI C ; NARDECCHIA F ; MAGNANI M**. Erythrocyte-mediated delivery of recombinant enzymes.. *J Inherit Metab Dis.*, 2016, vol. 39 (4), 519-20

• **MACHOVER D ; ROSSI L ; HAMELIN J ; DESTERKE C ; GOLDSCHMIDT E ; CHADEFAUX-VEKEMANS B ; BONNARME P ; BRIOZZO P ; KOPECNY D ; PIERIGÈ F**. Effects in cancer cells of the recombinant L-methionine Gamma-lyase from Brevibacterium aurantiacum. Encapsulation in human erythrocytes for sustained L-methionine elimination. *J Pharmacol. Exp. Ther.*, 2019, vol. 369, 489-502

• **MAGNANI M ; ROSSI L ; BIANCHI M ; FORNAINI G ; BENATTI U ; GUIDA L ; ZOCCHI E ; DE FLORA A**. Improved metabolic properties of hexokinase-overloaded human erythrocytes.. *Biochim Biophys Acta*, 1988, vol. 972 (1), 1-8

• CRatine Deficiency Syndromes. **MERCIMEK-MAHMUTOGLU S ; SALOMONS GS**. GeneReviews. University of Washington, 2009

• **NEWMAN EB ; BUDMAN LI ; CHAN EC ; GREENE RC ; LIN RT ; WOLDRINGH CL ; D'ARI R**. Lack of S-adenosylmethionine results in a cell division defect in Escherichia coli. *J Bacteriol.*, 1998, vol. 180 (14), 3614-9

• **ROSSI L** ; **PIERIGÈ F** ; **ALIANO MP** ; **MAGNANI M**. Ongoing developments and clinical progress in drug-loaded red blood cells technologies. *BioDrugs.*, 2020, vol. 34, 265-272

• **SHIOZAKI S** ; **SHIMIZU C** ; **YAMADA H**. Unusual Intracellular Accumulation of S-Adenosyl-L-methionine by Microorganisms. *Agric. Biol. Chem.*, 1984, vol. 48 (9), 2293-2300

• **WANG W** ; **KRAMER PM** ; **YANG S** ; **PEREIRA MA** ; **TAO L**. Reversed-phase high-performance liquid chromatography procedure for the simultaneous determination of S-adenosyl-L-methionine and S-adenosyl-L-homocysteine in mouse liver and the effect of methionine on their concentrations.. *J. Chromatogr. B Biomed. Sci. Appl.*, 2001, vol. 762 (1), 59-65

• **WISE CK** ; **COONEY CA** ; **ALI SF** ; **POIRIER LA**. Measuring S-adenosylmethionine in whole blood, red blood cells and cultured cells using a fast preparation method and high-performance liquid chromatography.. *J. Chromatogr. B Biomed. Sci. Appl.*, 1997, vol. 696 (1), 145-152